# EUROPÄISCHE PATENTSCHRIFT

(11) **EP 4 471 710 B1**
(45) Veröffentlichungstag und Bekanntmachung des Hinweises auf die Patenterteilung: **17.12.2025**
(21) Anmeldenummer: 23176138.8
(22) Anmeldetag: 30.05.2023
(51) Int. Cl.: G06T 7/00, G06T 7/90, G06N 3/02

(54) **ERKENNEN VON ARTEFAKTEN IN SYNTHETISCHEN MEDIZINISCHEN AUFNAHMEN**
DETECTION OF ARTIFACTS IN SYNTHETIC MEDICAL RECORDS
DÉTECTION D'ARTÉFACTS DANS DES ENREGISTREMENTS MÉDICAUX SYNTHÉTIQUES

(43) Veröffentlichungstag der Anmeldung: 04.12.2024
(73) Patentinhaber: Bayer Aktiengesellschaft, 51373 Leverkusen (DE)
(72) Erfinder: LENGA, Matthias, 51373 Leverkusen (DE); BALTRUSCHAT, Ivo Matteo, 51373 Leverkusen (DE)
(74) Vertreter: BIP Patents

(56) Entgegenhaltungen:
- WO-A1-2022/020531
- WO-A1-2022/220721
- US-A1- 2019 122 073
- US-A1- 2019 188 852
- US-A1- 2019 220 977
- US-A1- 2019 333 219
- US-A1- 2022 088 410
- RICHARD OSUALA ET AL: "Data synthesis and adversarial networks: A review and meta-analysis in cancer imaging", ARXIV.ORG, CORNELL UNIVERSITY LIBRARY, 201 OLIN LIBRARY CORNELL UNIVERSITY ITHACA, NY 14853, 27 November 2022 (2022-11-27), XP091378925, DOI: 10.1016/J.MEDIA.2022.102704
- SAMANEH KAZEMIFAR ET AL: "MRI-only brain radiotherapy: assessing the dosimetric accuracy of synthetic CT images generated using a deep learning approach", ARXIV.ORG, CORNELL UNIVERSITY LIBRARY, 201 OLIN LIBRARY CORNELL UNIVERSITY ITHACA, NY 14853, 11 April 2019 (2019-04-11), XP081168148, DOI: 10.1016/J.RADONC.2019.03.026

## Beschreibung

### HINWEIS ZUM URHEBERRECHT

Ein Teil der Offenbarung dieser Patentschrift enthält Material, das dem Urheberrechtsschutz unterliegt. Der Urheberrechtsinhaber hat keine Einwände gegen die Faksimile-Reproduktion der Patentschrift, wie sie in einer Patentake oder in den Akten des Patentamts erscheint, behält sich aber ansonten alle Urheberrechte und Rechte jeglicher Art vor. Ⓒ 2023 Bayer AG

### TECHNISCHES GEBIET

Die vorliegende Offenbarung betrifft das technische Gebiet der Erzeugung von synthetischen medizinischen Aufnahmen. Gegenstände der vorliegenden Offenbarung sind ein Verfahren, ein Computersystem und ein computerlesbares Speichermedium umfassend ein Computerprogramm zum Erkennen von Artefakten in synthetischen medizinischen Aufnahmen.

### EINLEITUNG

Künstliche Intelligenz findet zunehmend Einzug in die Medizin. Modelle des maschinellen Lernens werden nicht nur verwendet, um Anzeichen von Krankheiten in medizinischen Aufnahmen des menschlichen oder tierischen Körpers zu identifizieren (siehe z.B. WO2018202541A1, WO2020229152A1), sie werden zunehmend auch eingesetzt, um synthetische (künstliche) medizinische Aufnahmen zu erzeugen.

WO2021052896A1 und WO2021069338A1 beschreiben beispielsweise Verfahren zum Erzeugen einer künstlichen medizinischen Aufnahme, die einen Untersuchungsbereich eines Untersuchungsobjekts in einer ersten Zeitspanne zeigt. Die künstliche medizinische Aufnahmen wird mit Hilfe eines trainierten Modells des maschinellen Lernens auf Basis von medizinischen Aufnahmen, die den Untersuchungsbereich in einer zweiten Zeitspanne zeigen, erzeugt. Mit Hilfe des Verfahrens können beispielsweise radiologische Untersuchungen beschleunigt werden; anstatt radiologische Aufnahmen über eine längere Zeitspanne zu messen, werden Messungen nur innerhalb eines Teils der Zeitpanne gemessen und eine oder mehrere radiologische Aufnahmen für den übrigen Teil der Zeitspanne mit Hilfe des trainierten Modells vorhergesagt.

WO2019/074938A1 und WO2022184297A1 beschreiben beispielsweise Verfahren zum Erzeugen einer künstlichen radiologischen Aufnahme, die einen Untersuchungsbereich eines Untersuchungsobjekts nach der Applikation einer Standardmenge eines Kontrastmittels zeigen, obwohl nur eine geringere Menge an Kontrastmittel als die Standardmenge appliziert worden ist. Die Standardmenge ist die vom Hersteller und/oder Vertreiber des Kontrastmittels empfohlene und/oder die von einer Zulassungsbehörde zugelassene und/oder die in einem Beipackzettel zum Kontrastmittel aufgeführte Menge. Die in WO2019/074938A1 und WO2022184297A beschriebenen Verfahren können also zur Reduzierung der Menge an Kontrastmittel eingesetzt werden.

Die von den trainierten Modellen des maschinellen Lernens erzeugten medizinischen Aufnahmen können Fehler aufweisen (siehe z.B.: K. Schwarz *et al.: On the Frequency Bias of Generative Models,* https://doi.org/10.48550/arXiv.2111.02447).

Solche Fehler können problematisch sein, da ein Arzt auf Basis der künstlichen medizinischen Aufnahmen eine Diagnose stellen und/oder eine Therapie initiieren könnte. Wenn ein Arzt künstliche medizinische Aufnahmen begutachtet, muss der Arzt wissen, ob Merkmale in den künstlichen medizinischen Aufnahmen auf reale Merkmale des Untersuchungsobjekts zurückgeführt werden können, oder ob es sich um Artefakte handelt, die auf Fehler bei der Vorhersage durch das trainierte Modell des maschinellen Lernens zurückzuführen sind.

Die Dokumente US 2019/188852 A1, US 2019/333219 A1 und Richard Osuala et al.: "Data synthesis and adversarial networks: A review and meta-analysis in cancer imaging" offenbaren weiteren Stand der Technik.

### ZUSAMMENFASSUNG

Die Erfindung ist in den beigefügten Ansprüchen definiert.

Diese und weitere Probleme werden durch die Gegenstände der vorliegenden Offenbarung adressiert.

Ein erster Gegenstand der vorliegenden Offenbarung ist ein computer-implementiertes Verfahren zum Erzeugen mindestens eines Vertrauenswertes für ein synthetisches Bild umfassend die Schritte:
- Empfangen mindestens eines Bildes eines Untersuchungsbereichs eines Untersuchungsobjekts,
- Erzeugen einer Vielzahl an Teilbildern auf Basis des mindestens einen empfangenen Bildes, wobei jedes Teilbild einen Teilbereich des Untersuchungsbereichs des Untersuchungsobjekts repräsentiert, wobei sich Teilbereiche, die von verschiedenen Teilbildern repräsentiert werden, teilweise aber nicht vollständig überlappen.
- für jedes erzeugte Teilbild: Erzeugen eines synthetischen Teilbildes zumindest anteilig auf Basis des erzeugten Teilbildes,
- Ermitteln von Farbwerten korrespondierender Bildelemente synthetischer Teilbilder, wobei korrespondierende Bildelemente denselben Teilbereich des Untersuchungsbereichs repräsentieren,
- Ermitteln eines Streuungsmaßes der Farbwerte korrespondierender Bildelemente,
- Ermitteln eines Vertrauenswertes auf Basis des Streuungsmaßes,
- Ausgeben des Vertrauenswertes.

Ein weiterer Gegenstand der vorliegenden Offenbarung ist ein Computersystem umfassend
einen Prozessor und
einen Speicher, der ein Anwendungsprogramm speichert, das so konfiguriert ist, dass es, wenn es vom Prozessor ausgeführt wird, eine Operation durchführt, wobei die Operation umfasst:
   - Empfangen mindestens eines Bildes eines Untersuchungsbereichs eines Untersuchungsobjekts,
   - Erzeugen einer Vielzahl an Teilbildern auf Basis des mindestens einen empfangenen Bildes, wobei jedes Teilbild einen Teilbereich des Untersuchungsbereichs des Untersuchungsobjekts repräsentiert, wobei sich Teilbereiche, die von verschiedenen Teilbildern repräsentiert werden, teilweise aber nicht vollständig überlappen.
   - für jedes erzeugte Teilbild: Erzeugen eines synthetischen Teilbildes zumindest anteilig auf Basis des erzeugten Teilbildes,
   - Ermitteln von Farbwerten korrespondierender Bildelemente synthetischer Teilbilder, wobei korrespondierende Bildelemente denselben Teilbereich des Untersuchungsbereichs repräsentieren,
   - Ermitteln eines Streuungsmaßes der Farbwerte korrespondierender Bildelemente,
   - Ermitteln eines Vertrauenswertes auf Basis des Streuungsmaßes,
   - Ausgeben des Vertrauenswertes.

Ein weiterer Gegenstand der vorliegenden Offenbarung ist ein computerlesbares Speichermedium umfassend ein Computerprogramm, das in einen Arbeitsspeicher eines Computersystems geladen werden kann und dort das Computersystem dazu veranlasst, folgende Schritte ausführen:
- Empfangen mindestens eines Bildes eines Untersuchungsbereichs eines Untersuchungsobjekts,
- Erzeugen einer Vielzahl an Teilbildern auf Basis des mindestens einen empfangenen Bildes, wobei jedes Teilbild einen Teilbereich des Untersuchungsbereichs des Untersuchungsobjekts repräsentiert, wobei sich Teilbereiche, die von verschiedenen Teilbildern repräsentiert werden, teilweise aber nicht vollständig überlappen.
- für jedes erzeugte Teilbild: Erzeugen eines synthetischen Teilbildes zumindest anteilig auf Basis des erzeugten Teilbildes,
- Ermitteln von Farbwerten korrespondierender Bildelemente synthetischer Teilbilder, wobei korrespondierende Bildelemente denselben Teilbereich des Untersuchungsbereichs repräsentieren,
- Ermitteln eines Streuungsmaßes der Farbwerte korrespondierender Bildelemente,
- Ermitteln eines Vertrauenswertes auf Basis des Streuungsmaßes,
- Ausgeben des Vertrauenswertes.

### KURZE BESCHREIBUNG DER ZEICHNUNGEN

Fig. 1 zeigt beispielhaft und schematisch das Erzeugen von Teilbildern auf Basis eines empfangenen Bildes eines Untersuchungsbereichs eines Untersuchungsobjekts.
Fig. 2 zeigt beispielhaft und schematisch das Erzeugen von synthetischen Teilbildern auf Basis von Teilbildern mit Hilfe eines generativen Modells und das Zusammenfügen der synthetischen Teilbilder zu einem synthetischen Bild.
Fig. 3 beispielhaft und schematisch das Kombinieren von synthetischen Bildern zu einem vereinten synthetischen Bild.
Fig. 4 zeigt beispielhaft und schematisch das Kombinieren von synthetischen Bildern zu einem vereinten synthetischen Bild.
Fig. 5a zeigt beispielhaft und schematisch, wie aus jedem Bild einer Mehrzahl an empfangenen Bildern Teilbilder erzeugt und auf Basis der erzeugten Teilbilder synthetische Teilbilder erzeugt werden.
Fig. 5b zeigt beispielhaft und schematisch, wie synthetische Teilbilder zu synthetischen Bildern zusammengefügt und wie die synthetischen Bilder zu einem vereinten synthetischen Bild kombiniert werden können.
Fig. 6 zeigt beispielhaft und schematisch das Ermitteln eines Vertrauenswertes.
Fig. 7 zeigt eine Ausführungsform des Verfahrens der vorliegenden Offenbarung in Form eines Ablaufschemas.
Fig. 8 zeigt beispielhaft und schematisch ein Verfahren zum Trainieren eines generativen Modell des maschinellen Lernens.
Fig. 9 zeigt beispielhaft und schematisch ein Computersystem gemäß der vorliegenden Offenbarung.
Fig. 10 zeigt beispielhaft und schematisch eine weitere Ausführungsform des Computersystems der vorliegenden Offenbarung.

### AUSFÜHRLICHE BESCHREIBUNG

Die Erfindung wird im Folgenden näher erläutert, ohne zwischen den Gegenständen der vorliegenden Offenbarung (Verfahren, Computersystem, computerlesbares Speichermedium) zu unterscheiden. Vielmehr sollen die nachfolgenden Ausführungen sinngemäß für alle Gegenstände der Erfindung gelten, unabhängig davon, in welchem Zusammenhang (Verfahren, Computersystem, computerlesbares Speichermedium) sie beschrieben werden.

Wenn in der vorliegenden Beschreibung oder in den Ansprüchen Schritte in einer Reihenfolge angegeben sind, bedeutet dies nicht unbedingt, dass die Erfindung auf die angegebene Reihenfolge beschränkt ist. Vielmehr ist es denkbar, dass die Schritte auch in einer anderen Reihenfolge oder auch parallel zueinander ausgeführt werden können, es sei denn, ein Schritt baut auf einem anderen auf, was zwingend erfordert, dass der aufbauende Schritt anschließend ausgeführt wird (dies wird aber im Einzelfall klar). Die genannten Reihenfolgen sind somit bevorzugte Ausführungsformen der vorliegenden Offenbarung.

Die Erfindung wird an einigen Stellen in Bezug auf Zeichnungen näher erläutert. Dabei sind in den Zeichnungen konkrete Ausführungsformen mit konkreten Merkmalen und Merkmalskombinationen dargestellt, die in erster Linie der Veranschaulichung dienen; die Erfindung soll nicht so verstanden werden, dass sie auf die in den Zeichnungen dargestellten Merkmale und Merkmalskombinationen beschränkt ist. Ferner sollen Aussagen, die bei der Beschreibung der Zeichnungen in Bezug auf Merkmale und Merkmalskombinationen getroffen werden, allgemein gelten, das heißt auch auf andere Ausführungsformen übertragbar und nicht auf die gezeigten Ausführungsformen beschränkt sein.

Die vorliegende Offenbarung beschreibt Mittel zum Beurteilen der Vertrauenswürdigkeit eines synthetischen Bildes eines Untersuchungsbereichs eines Untersuchungsobjekts.

Unter dem Begriff "Vertrauenswürdigkeit" wird verstanden, dass eine Person, die das synthetische Bild begutachtet, darauf vertrauen kann, dass Strukturen und/oder Morphologien und/oder Texturen, die in dem synthetischen Bild dargestellt sind, auf reale Strukturen und/oder reale Morphologien und/oder reale Texturen des Untersuchungsbereichs des Untersuchungsobjekts zurückgeführt werden können und keine Artefakte sind.

Der Begriff "synthetisch" bedeutet, dass das synthetische Bild nicht das unmittelbare Ergebnis einer Messung an einem realen Untersuchungsobjekt ist, sondern künstlich erzeugt (berechnet) wurde. Dabei kann ein synthetisches Bild jedoch auf Bildaufnahmen eines realen Untersuchungsobjekts basieren, d.h. es können ein oder mehrere Bildaufnahmen eines realen Untersuchungsobjekts verwendet werden, um das synthetische Bild zu erzeugen. In der Einleitung und in der weiteren Beschreibung der vorliegenden Offenbarung sind Beispiele für synthetische Bilder beschrieben.

Das "Untersuchungsobjekt" ist vorzugsweise ein Mensch oder ein Tier, vorzugsweise ein Säugetier, ganz besonders bevorzugt ein Mensch.

Der "Untersuchungsbereich" ist ein Teil des Untersuchungsobjekts, zum Beispiel ein Organ eines Menschen oder Tieres wie beispielsweise die Leber, das Gehirn, das Herz, die Niere, die Lunge, der Magen, der Darm, die Bauchspeicheldrüse, die Schilddrüse, die Prostata, die Brust oder ein Teil der genannten Organe oder mehrere Organe oder ein anderer Teil des Untersuchungsobjekts. Der Untersuchungsbereich kann auch mehre Organe und/oder Teile von mehreren Organen umfassen.

Der Begriff "Bild" bezeichnet eine Datenstruktur, die eine räumliche Verteilung eines physikalischen Signals darstellt. Die räumliche Verteilung kann eine beliebige Dimension haben, z.B. 2D, 3D, 4D oder eine höhere Dimension. Die räumliche Verteilung kann eine beliebige Form haben, z.B. ein Gitter bilden und dadurch Pixel oder Voxel definieren, wobei das Gitter unregelmäßig oder regelmäßig sein kann. Das physikalische Signal kann ein beliebiges Signal sein, z.B. Protonendichte, Echogenität, Durchlässigkeit, Absorptionsvermögen, Relaxivität, Informationen über rotierende Wasserstoffkerne in einem Magnetfeld, Farbe, Graustufe, Tiefe, Oberflächen- oder Volumenbelegung.

Unter dem Begriff "Bild" wird vorzugsweise eine zwei-, drei- oder höherdimensionale visuell erfassbare Repräsentation des Untersuchungsbereichs des Untersuchungsobjekts verstanden. Üblicherweise handelt es sich bei dem empfangenen Bild um ein digitales Bild. Der Begriff "digital" bedeutet, dass das Bild von einer Maschine, in der Regel einem Computersystem, verarbeitet werden kann. Unter "Verarbeitung" werden die bekannten Verfahren zur elektronischen Datenverarbeitung (EDV) verstanden.

Ein digitales Bild kann mit Computersystemen und Software verarbeitet, bearbeitet und reproduziert sowie in standardisierte Datenformate konvertiert werden, wie zum Beispiel JPEG (Grafikformat der Joint Photographic Experts Group), PNG (Portable Network Graphics) oder SVG (Scalable Vector Graphics). Digitale Bilder können mit geeigneten Anzeigegeräten visualisiert werden, wie zum Beispiel mit Computermonitoren, Projektoren und/oder Druckern.

In einem digitalen Bild werden Bildinhalte üblicherweise durch ganze Zahlen repräsentiert und gespeichert. In den meisten Fällen handelt es sich um zwei- oder dreidimensionale Bilder, die binär kodiert und gegebenenfalls komprimiert sein können. Bei den digitalen Bildern handelt es sich üblicherweise um Rastergrafiken, bei denen die Bildinformation in einer gleichmäßigen Rasterung abgelegt ist. Rastergrafiken bestehen aus einer rasterförmigen Anordnung von so genannten Bildpunkten (Pixel) im Fall von zweidimensionalen Darstellungen oder Volumenelementen (Voxel) im Fall dreidimensionaler Darstellungen. Bei vierdimensionalen Darstellungen wird häufig der Begriff Doxel *(dynamic voxel)* für die Bildelemente verwendet. Bei höherdimensionalen Darstellungen oder allgemein wird im Englischen manchmal auch der Begriff "*n*-xel" verwendet, wobei n die jeweilige Dimension angibt. In dieser Offenbarung wird allgemein der Begriff Bildelement verwendet. Ein Bildelement kann also ein Bildpunkt (Pixel) im Fall einer zweidimensionalen Darstellung, ein Volumenelement (Voxel) im Fall einer dreidimensionalen Darstellung, ein dynamisches Voxel (Doxel) im Fall der vierdimensionalen Darstellung oder ein höherdimensionales Bildelement im Fall einer höherdimensionalen Darstellung sein.

Jedem Bildelement eines Bildes ist ein Farbwert zugeordnet. Der Farbwert gibt an, wie (z.B. in welcher Farbe) das Bildelement visuell dargestellt werden soll (z.B. auf einem Monitor oder einem Drucker).

Der einfachste Fall ist ein Binärbild, bei dem ein Bildelement entweder weiß oder schwarz dargestellt wird. Üblicherweise steht der Farbwert "0" für "schwarz" und der Farbwert "1" für "weiß".

Bei einem Graustufenbild ist jedem Bildelement eine Graustufe zugeordnet, die von schwarz über eine definierte Zahl von Grauabstufungen bis weiß reicht. Die Graustufen werden auch als Grauwerte bezeichnet. Die Zahl der Abstufungen kann beispielsweise von 0 bis 255 reichen (also 256 Graustufen/Grauwerte umfassen), wobei auch hier der Wert "0" üblicherweise für "schwarz" steht und der höchste Grauwert (in dem vorliegenden Beispiel der Wert 255) für "weiß" steht.

Bei einem Farbbild definiert sich die für ein Bildelement verwendete Kodierung der Farbe unter anderem über den Farbraum und die Farbtiefe. Bei einem Bild, dessen Farbe über den so genannten RGB-Farbraum definiert ist (RGB steht für die Grundfarben Rot, Grün und Blau), sind jedem Bildpunkt drei Farbwerte zugeordnet, ein Farbwert für die Farbe Rot, ein Farbwert für die Farbe Grün und ein Farbwert für die Farbe Blau. Die Farbe eines Bildelements ergibt sich durch Überlagerung (additives Mischen) der drei Farbwerte. Der einzelne Farbwert kann z.B. in 256 unterscheidbare Stufen diskretisiert sein, die Tonwerte genannt werden und üblicherweise von 0 bis 255 reichen. Der Tonwert "0" eines jeden Farbkanals ist üblicherweise die dunkelste Farbnuance. Haben alle drei Farbkanäle den Tonwert 0, erscheint das entsprechende Bildelement schwarz; haben alle drei Farbkanäle den Tonwert 255, erscheint das entsprechende Bildelement weiß.

Unabhängig davon, ob es sich um ein Binärbild, ein Graustufenbild oder ein Farbbild handelt, wird in dieser Offenbarung der Begriff "Farbwert" für die Information verwendet, in welcher Farbe (inkl. den "Farben" "schwarz" und "weiß" sowie allen Grautönen) ein Bildelement dargestellt werden soll. Ein Farbwert kann also ein Tonwert eines Farbkanals sein, ein Grauton sein oder für "schwarz" oder für "weiß" stehen.

Ein Farbwert in einem Bild (insbesondere einer medizinischen Aufnahme) repräsentiert üblicherweise eine Stärke eine physikalischen Signals (s.o.). Es sei angemerkt, dass der Farbwert auch ein Wert für das physikalische Signal selbst sein kann.

Es gibt eine Vielzahl an möglichen digitalen Bildformaten und Farbkodierungen. Vereinfachend wird in dieser Beschreibung davon ausgegangen, dass die vorliegenden Bilder RGB-Rastergrafiken mit einer spezifischen Zahl an Bildelementen sind. Diese Annahme soll jedoch in keiner Weise limitierend verstanden werden. Dem Fachmann der Bildbearbeitung ist klar, wie er die Lehre dieser Beschreibung auf Bilddateien, die in anderen Bildformaten vorliegen und/oder bei denen die Farbwerte anders kodiert sind, übertragen kann.

Bei einem "Bild" im Sinne der vorliegenden Offenbarung kann es sich auch um einen oder mehrere Ausschnitte aus einer Videosequenz handeln.

In einem ersten Schritt wird mindestens ein Bild eines Untersuchungsbereichs eines Untersuchungsobjekts empfangen.

Der Begriff "Empfangen" umfasst sowohl das Abrufen von Bildern als auch das Entgegennehmen von Bildern, die z.B. an das Computersystem der vorliegenden Offenbarung übermittelt werden. Das mindestens eine Bild kann von einem Computertomografen, von einem Magnetresonanztomografen, von einem Ultraschall-Scanner, von einer Kamera und/oder von einem anderen Gerät zur Erzeugung von Bildern empfangen werden. Das mindestens eine Bild kann aus einem Datenspeicher ausgelesen und/oder von einem separaten Computersystem übermittelt werden.

Vorzugsweise handelt es sich bei dem mindestens einen empfangenen Bild um eine zweidimensionale oder dreidimensionale Repräsentation eines Untersuchungsbereichs eines Untersuchungsobjekts.

In einer Ausführungsform der vorliegenden Offenbarung handelt es sich bei dem mindestens einen empfangenen Bild um eine medizinische Aufnahme.

Eine "medizinische Aufnahme" ist eine visuelle Repräsentation eines Untersuchungsbereichs eines Menschen oder eines Tieres, die für diagnostische und/oder therapeutische Zwecke verwendet werden kann.

Es gibt eine Vielzahl an Techniken, mit denen medizinische Aufnahmen erzeugt werden können; Beispiele solcher Techniken sind Röntgen, Computertomografie (CT), Fluoroskopie, Magnetresonanztomografie (MRT), Ultraschall (Sonografie), Endoskopie, Elastografie, taktile Bildgebung, Thermografie, Mikroskopie, Positronenemissionstomografie, optische Kohärenztomografie (OCT), Fundusfotografie und andere.

Beispiele für medizinische Aufnahmen sind CT-Aufnahmen, Röntgenbilder, MRT-Aufnahmen, Fluoreszenzangiografie-Bilder, OCT-Aufnahmen, histologische Aufnahmen, Ultraschallbilder, Fundusbilder und/oder andere.

Das mindestens eine empfangene Bild kann eine CT-Aufnahme, MRT-Aufnahme, Ultraschallaufnahme, OCT-Aufnahme, und/oder eine andere Repräsentation eines Untersuchungsbereichs eines Untersuchungsobjekts sein.

Das mindestens eine empfangene Bild kann auch Repräsentationen unterschiedlicher Modalitäten umfassen, z.B. eine CT-Aufnahme und eine MRT-Aufnahme.

Jedes empfangene Bild umfasst eine Vielzahl an Bildelementen. Jedes Bildelement der Vielzahl an Bildelementen repräsentiert einen Teilbereich des Untersuchungsbereichs des Untersuchungsobjekts. Der Begriff "Vielzahl an Bildelementen" bedeutet mindestens 1000, vorzugsweise mindestens 10000, noch mehr bevorzugt mehr als 100000. Es ist denkbar, dass ein empfangenes Bild ein oder mehrere Bildelemente umfasst, die nicht den Untersuchungsbereich des Untersuchungsobjekts repräsentieren, sondern einen anderen Bereich wie beispielsweise einen angrenzenden und/oder umgebenden Bereich.

In einem ersten Schritt wird auf Basis des mindestens einen Bildes eine Vielzahl an Teilbildern erzeugt. Der Begriff "Vielzahl an Teilbildern" bedeutet mindestens zwei, vorzugsweise mindestens zehn, noch mehr bevorzugt mehr als zwanzig Teilbilder.

Jedes Teilbild repräsentiert einen Teilbereich des Untersuchungsbereichs des Untersuchungsobjekts. Teilbereiche, die von verschiedenen Teilbildern repräsentiert werden, überlappen sich teilweise aber nicht vollständig. Mit anderen Worten: es gibt mindestens zwei Teilbilder, die jeweils einen Teilbereich des Untersuchungsbereichs repräsentieren, wobei sich die Teilbereiche zum Teil überlappen, aber nicht vollständig überlappen. Mit anderen Worten: es gibt Teilbereiche des Untersuchungsbereichs, die von mehreren (mindestens zwei) Teilbildern repräsentiert werden, wobei die Teilbilder, die denselben Teilbereich repräsentieren, verschiedene weitere Teilbereich repräsentieren.

Vorzugsweise wird jeder Teilbereich des Untersuchungsbereichs von mehreren (mindestens zwei) Teilbildern in unterschiedlichen Konstellationen mit anderen Teilbereichen repräsentiert.

Ein Teilbereich des Untersuchungsbereichs wird durch ein oder mehrere Bildelemente eines Teilbildes repräsentiert. Es gibt also mindestens zwei Teilbilder, die mindestens ein Bildelement gemeinsam haben, sich aber in mindestens einem Bildelement unterscheiden.

Vorzugsweise gibt es für jedes Teilbild mindestens ein anderes Teilbild mit mindestens einem gemeinsamen Bildelement und mindestens einem unterschiedlichen Bildelement. Noch mehr bevorzugt gibt es für jedes Teilbild mehrere andere Teilbilder mit mindestens einem gemeinsamen Bildelement und mindestens einem unterschiedlichen Bildelement.

Vorzugsweise ist die Zahl der gleichen (gemeinsamen) Bildelemente und/oder der unterschiedlichen Bildelemente größer als 10, noch mehr bevorzugt größer als 100.

Vorzugsweise gibt es zu jedem Bildelement des mindestens einen empfangenen Bildes eine Mehrzahl an Teilbildern, die dieses Bildelement ebenfalls umfassen, wobei sich jedes Teilbild der Mehrzahl an Teilbildern von jedem anderen Teilbild der Mehrzahl an Teilbildern durch mindestens ein anderes Bildelement unterscheidet.

Die Teilbilder können beispielsweise dadurch erzeugt werden, dass das mindestens eine empfangene Bild durch Schnitte in Teilbilder aufgeteilt wird, wobei die Schnittlinien (bei 2D-Bildern) bzw. Schnittflächen (bei 3D-Bilder) in unterschiedlichen Winkeln durch das mindestens eine empfangene Bild laufen.

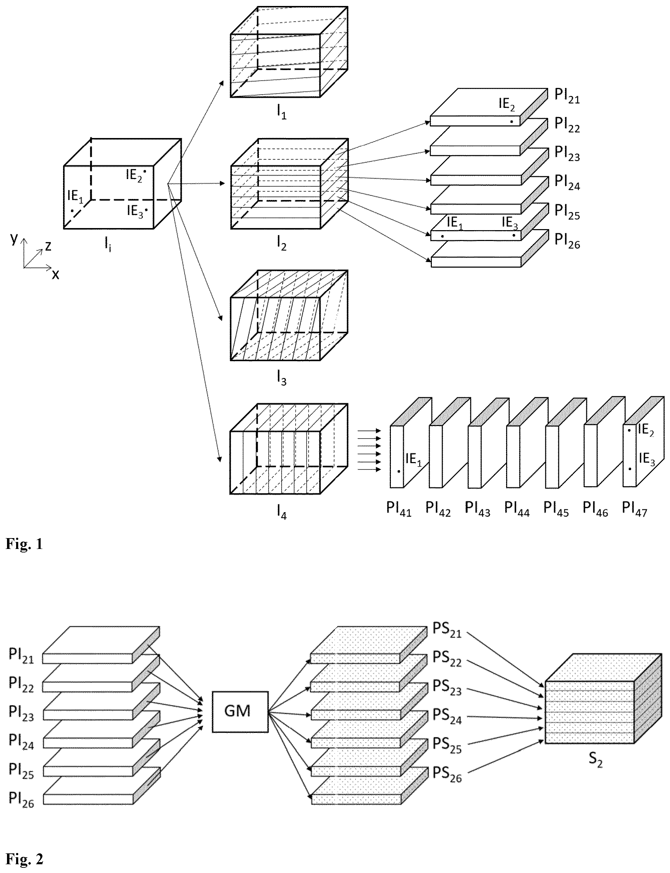

Eine Ausführungsform zum Erzeugen von Teilbildern wird nachfolgend anhand von Fig. 1 näher erläutert, ohne die Erfindung auf die in Fig. 1 gezeigte Ausführungsform beschränken zu wollen. Aussagen, die zu den in den Zeichnungen der vorliegenden Offenbarung dargestellten Ausführungsformen getroffen werden, sollen in analoger Weise auch für alle anderen Ausführungsformen gelten.

Fig. 1 zeigt ein empfangenes Bild Iᵢ eines Untersuchungsbereichs eines Untersuchungsobjekts. Das empfangene Bild Iᵢ umfasst eine Vielzahl an Bildelementen, drei Bildelemente IE₁, IE₂ und IE₃ sind als Punkte dargestellt.

Von dem empfangenen Bild Iᵢ wird eine Vielzahl an Kopien I₁, I₂, I₃ und I₄ erzeugt. Eine der Kopien I₁, I₂, I₃ oder I₄ kann das empfangene Bild Iᵢ selbst sein. Die Zahl der Kopien, die pro empfangenem Bild erzeugt werden, entspricht üblicherweise der Zahl der synthetischen Bilder, die erzeugt und dann zu einem vereinten synthetischen Bild kombiniert werden können.

Jede Kopie I₁, I₂, I₃ und I₄ wird in eine Vielzahl an Teilbildern unterteilt. Dies erfolgt im vorliegenden Beispiel dadurch, dass jede Kopie durch Schnitte entlang von Schnittebenen unterteilt wird. Dabei verlaufen die Schnittebenen in unterschiedlichen Winkeln durch die Kopien I₁, I₂, I₃ und I₄. Im Fall der Kopie I₂ verlaufen die Schnittebenen beispielsweise parallel zur xz-Ebene; es entstehen die Teilbilder PI₂₁, PI₂₂, PI₂₃, PI₂₄, PI₂₅ und PI₂₆. Im Fall der Kopie I₄ verlaufen die Schnittebenen beispielsweise parallel zur yz-Ebene; es entstehen die Teilbilder PI₄₁, PI₄₂, PI₄₃, PI₄₄, PI₄₅, PI₄₆ und PI₄₇. Die Aufteilung der Kopien I₁ und I₃ ist in Fig. 1 nur angedeutet; es sind Schnittebenen dargestellt; die aus den entsprechenden Schnitten entlang der Schnittebenen resultierenden Teilbilder sind in Fig. 1 nicht explizit dargestellt.

Im vorliegenden Beispiel werden die Kopien durch ebene Flächen in Teilbilder unterteilt. Dies ist eine bevorzugte Ausführungsform der vorliegenden Erfindung. Es ist jedoch auch möglich, die Kopien durch gekrümmte Flächen oder durch andere Schnitte in Teilbilder zu unterteilen.

Im vorliegenden Beispiel haben die Schnittebenen bei den einzelnen Kopien den gleichen Abstand voneinander. Dies ist eine bevorzugte Ausführungsform der vorliegenden Erfindung. Es ist aber auch möglich, dass der Abstand der Schnittebenen (oder allgemein der Schnittflächen) einen variierenden Abstand voneinander aufweisen.

Im Fall der Kopien I₂ und I₄ haben alle Teilbilder dieselbe Größe (sie haben die gleiche Zahl an Bildelementen). Mit anderen Worten: der Teilbereich des Untersuchungsbereichs, den sie jeweils repräsentieren, ist für alle Teilbilder gleich groß. Im Fall der Kopien I₁ und I₃ hat nur ein Teil der Teilbilder dieselbe Größe. Es ist möglich, alle Teilbilder auf dieselbe Größe zu bringen, in dem Bereiche in Teilbilder mit geringerer Größe durch Padding mit Nullen (oder einem anderen Wert) aufgefüllt werden.

Die Zahl der Teilbilder, die aus den einzelnen Kopien erzeugt wird, kann gleich oder unterschiedlich sein. Im vorliegenden Beispiel variiert die Zahl an Teilbildern, die aus den einzelnen Kopien erzeugt wird. Im Fall der Kopie I₁ sind es sechs, im Fall der Kopie I₂ sind es sechs, im Fall der Kopie I₃ sind es neun und Fall der Kopie I₄ sind es sieben.

Vorzugsweise erfolgt die Aufteilung der Kopien in Teilbilder so, dass alle entstehenden Teilbilder dieselbe Größe (d.h. Anzahl an Bildelementen) haben; wobei dies im Einzelfall durch Padding erreicht werden kann. Dies hat den Vorteil, dass dem generativen Model stets Teilbilder derselben Größe zugeführt werden können.

Die in Fig. 1 dargestellte Erzeugung von Teilbildern erfüllt die oben genannten Voraussetzungen:
- Es gibt mindestens zwei Teilbilder, die denselben Teilbereich des Untersuchungsbereichs des Untersuchungsobjekts repräsentieren, die aber zusätzlich jeweils einen anderen Teilbereich repräsentieren: Der Teilbereich, der durch das Bildelement IE₁ repräsentiert wird, wird sowohl durch das Teilbild PI₂₅ als auch durch das Teilbild PI₄₁ repräsentiert, jedes der Teilbilder PI₂₅ und PI₄₁ repräsentiert jedoch zusätzlich noch andere Teilbereiche des Untersuchungsbereichs, die durch das jeweils andere Teilbild nicht repräsentiert. So repräsentiert das Teilbild PI₂₅ beispielsweise mit dem Bildelement IE₃ einen Teilbereich, der durch das Teilbild PI₄₁ nicht repräsentiert wird. Das Teilbild P₄₇ repräsentiert mit dem Bildelement IE₃ einen Teilbereich, der auch durch das Teilbild PI₂₅ repräsentiert wird; es repräsentiert aber mit dem Bildelement IE₂ einen Teilbereich, der durch das Teilbild PI₂₅ nicht repräsentiert.
- Die Teilbilder PI₂₅ und PI₄₇ haben das Bildelement IE₃ gemeinsam, unterscheiden sich aber in mindestens einem anderen Bildelement: Teilbild PI₂₅ umfasst beispielsweise das Bildelement IE₁, das das Teilbild PI₄₇ nicht umfasst und das Teilbild PI₄₇ umfasst das Bildelement IE₂, das das Teilbild PI₂₅ nicht umfasst.

Die in Fig. 1 dargestellte Erzeugung von Teilbildern erfüllt ferner eine weitere Bedingung einer oben genannten Ausführungsform:
- Es gibt für jedes Teilbild mindestens ein anderes Teilbild mit mindestens einem gemeinsamen Bildelement und mindestens einem unterschiedlichen Bildelement. Im vorliegenden Beispiel gibt es für jedes Teilbild mindestens drei Teilbilder mit mindestens einem gemeinsamen Bildelement und mindestens einem unterschiedlichen Bildelement.

In einem nächsten Schritt werden auf Basis der Teilbilder synthetische Teilbilder erzeugt. Die Erzeugung der synthetischen Teilbilder erfolgt mittels eines Modells, das in dieser Offenbarung als generatives Modell bezeichnet wird.

Das generative Modell kann ein trainiertes Modell des maschinellen Lernens sein. Ein "Modell des maschinellen Lernens" kann als eine computerimplementierte Datenverarbeitungsarchitektur verstanden werden. Ein solches Modell kann Eingabedaten empfangen und Ausgabedaten auf der Grundlage dieser Eingabedaten und Modellparametern liefern. Ein solches Modell kann durch Training eine Beziehung zwischen den Eingabedaten und den Ausgabedaten erlernen. Beim Training können Modellparameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modellparameter werden so verändert, dass die Abweichungen zwischen den Ausgabedaten und den Zieldaten auf ein (definiertes) Minimum reduziert werden. Zur Modifizierung der Modellparameter im Hinblick auf eine Reduzierung der Abweichungen kann ein Optimierungsverfahren wie beispielsweise ein Gradientenverfahren verwendet werden.

Die Abweichungen können mit Hilfe einer Fehlerfunktion (engl.: *loss function)* quantifiziert werden. Eine solche Fehlerfunktion kann verwendet werden, um einen Fehler (engl.: *loss)* für ein gegebenes Paar von Ausgabedaten und Zieldaten zu berechnen. Das Ziel des Trainingsprozesses kann darin bestehen, die Parameter des Modells des maschinellen Lernens so zu verändern (anzupassen), dass der Fehler für alle Paare des Trainingsdatensatzes auf ein (definiertes) Minimum reduziert wird.

Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Fehler bedeuten, dass ein oder mehrere Modellparameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder eine andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

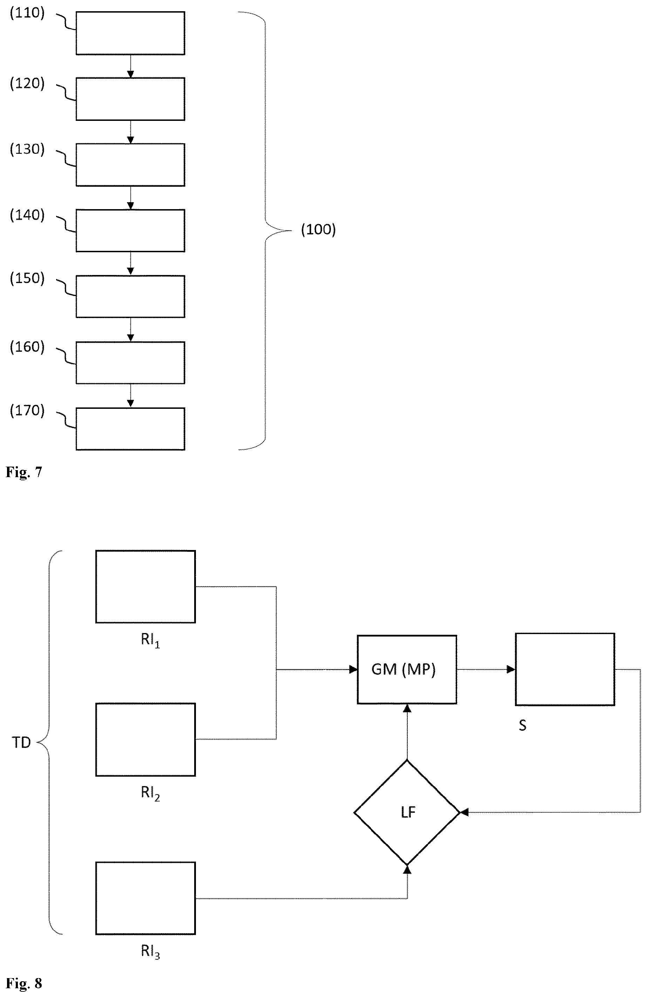

Fig. 8 zeigt schematisch ein Beispiel für das Trainieren eines Modells des maschinellen Lernen und ist weiter unten näher beschrieben.

Das generative Modell kann einen oder mehrere Algorithmen umfassen, die angeben, wie ein synthetisches Teilbild auf Basis eines oder mehrerer Teilbilder erzeugt werden kann. Üblicherweise werden ein oder mehrere Teilbilder dem generativen Modell zugeführt und das Modell erzeugt auf Basis des einen oder der mehreren Teilbilder, Modellparametern und gegebenenfalls weiteren Eingabedaten ein synthetisches Teilbild.

Das generative Modell kann ein Modell des maschinellen Lernens sein, wie es beispielsweise in einer der folgenden Publikationen beschrieben ist: WO2019/074938A1, WO2022/253687A1, WO2022/207443A1, WO2022/223383A1, WO20227184297A1, WO2022/179896A2, WO2021/069338A1, EP 22209510.1, EP23159288.2, PCT/EP2023/053324, PCT/EP2023/050207, CN110852993A, CN110853738A, US2021150671A1, arXiv:2303.15938v1, doi:10.1093/jrr/rrz030.

Das generative Modell kann beispielsweise konfiguriert sein, auf Basis mindestens einer empfangenen radiologischen Aufnahme des Untersuchungsbereichs vor und/oder nach einer Applikation einer ersten Menge eines Kontrastmittels, eine synthetische radiologische Aufnahme nach der Applikation einer zweiten Menge des Kontrastmittels zu erzeugen, wobei die zweite Menge vorzugsweise größer als die erste Menge ist (wie z.B. beschrieben in WO2019/074938A1 oder WO2022184297A1).

Die mindestens eine empfangene radiologische Aufnahme kann beispielsweise eine MRT-Aufnahme sein, und die synthetische radiologische Aufnahme kann eine synthetische MRT-Aufnahme sein.

Die mindestens eine empfangene radiologische Aufnahme kann auch eine CT-Aufnahme vor und/oder nach der Applikation einer ersten Menge eines MRT-Kontrastmittels umfassen, und die synthetische radiologische Aufnahme kann eine synthetische CT-Aufnahme nach der Applikation einer zweiten Menge eines MRT-Kontrastmittels sein, wobei die zweite Menge vorzugsweise größer als die erste Menge und vorzugsweise größer als die Standardmenge des MRT-Kontrastmittels für MRT-Untersuchungen ist (wie z.B. beschrieben in PCT/EP2023/053324). Die "Standardmenge" ist üblicherweise die vom Hersteller und/oder Vertreiber des Kontrastmittels empfohlene und/oder die von einer Zulassungsbehörde zugelassene und/oder die in einem Beipackzettel zum Kontrastmittel aufgeführte Menge.

Das generative Modell kann beispielsweise konfiguriert sein, auf Basis mindestens einer radiologischen Aufnahme eines Untersuchungsbereichs eines Untersuchungsobjekts, die den Untersuchungsbereich in einer ersten Zeitspanne vor/und oder nach der Applikation eines Kontrastmittels repräsentiert, eine synthetische radiologische Aufnahme zu erzeugen, die den Untersuchungsbereich in einer zweiten Zeitspanne nach der Applikation des Kontrastmittels repräsentiert (wie z.B. beschrieben in WO2021052896A1).

Im Gegensatz zu den Verfahren, die in den oben zitierten Publikationen beschrieben sind, wird im Fall der vorliegenden Offenbarung jedoch nicht nur ein synthetisches Bild erzeugt, sondern es wird eine Mehrzahl an synthetischen Bildern erzeugt, die in einem nachfolgenden Schritt zu einem einzigen synthetischen Bild - in dieser Beschreibung als vereintes synthetisches Bild bezeichnet - kombiniert werden können.

Der Begriff "Mehrzahl an synthetischen Bildern" bedeutet mindestens zwei, vorzugsweise mindestens fünf, noch mehr bevorzugt mindestens zehn synthetische Bilder.

Dabei unterscheiden sich die synthetischen Bilder der Mehrzahl an synthetischen Bildern dadurch voneinander, dass sie zumindest teilweise auf Basis unterschiedlicher Teilbilder des mindestens einen empfangenen Bildes erzeugt werden. Teilbilder unterscheiden sich in der Konstellation der Bildelemente, aus denen sie sich zusammensetzen; unterschiedliche Teilbilder können die gleichen Bildelemente aufweisen, sie weisen solche gleichen Bildelemente aber in unterschiedlichen Konstellationen mit anderen Bildelementen auf.

Mit anderen Worten wird jedes synthetische Bild der Mehrzahl an synthetischen Bildern auf Basis von Teilbildern erzeugt, die eine unterschiedliche Konstellation an Bildelementen aufweisen. Dem generativen Modell werden also teilweise unterschiedliche Eingabedaten zugeführt; mit Hilfe des generativen Modells wird auf Basis der teilweise unterschiedlichen Eingabedaten die Mehrzahl an synthetischen Bildern erzeugt.

Aus den Unterschieden zwischen den synthetischen Bildern der Mehrzahl an synthetischen Bildern lässt sich dann ein Vertrauenswert ermitteln, der angibt, wie vertrauenswürdig ein vereintes synthetisches Bild, das durch Kombination der Mehrzahl an synthetischen Bildern gewonnen werden kann, ist. Details zur Ermittlung des Vertrauenswerts sind weiter unten in der Beschreibung beschrieben.

Fig. 2 zeigt beispielhaft und schematisch die Erzeugung von synthetischen Teilbildern auf Basis von Teilbildern mit Hilfe eines generativen Modells und das Zusammenfügen der synthetischen Teilbilder zu einem synthetischen Bild. Bei den Teilbildern handelt es sich um die in Fig. 1 dargestellten Teilbilder PI₂₁, PI₂₂, PI₂₃, PI₂₄, PI₂₅ und PI₂₆.

Auch wenn Fig. 2 den Eindruck erwecken mag, dass die Teilbilder PI₂₁, PI₂₂, PI₂₃, PI₂₄, PI₂₅ und PI₂₆ aus Fig. 1 dem generativen Modell GM gemeinsam zugeführt werden, werden die Teilbilder PI₂₁, PI₂₂, PI₂₃, PI₂₄, PI₂₅ und PI₂₆ dem generativen Modell GM separat voneinander (z.B. nacheinander) zugeführt. Das generative Modell GM ist konfiguriert, auf Basis eines Teilbildes PI₂ₘ, eine synthetische Teil-Repräsentation PS₂ₘ zu erzeugen, wobei m ein Index ist, der im vorliegenden Beispiel die Ganzzahlen 1 bis 6 durchläuft. Im vorliegenden Beispiel wird also auf Basis des Teilbildes PI₂₁ das synthetische Teilbild PS₂₁ erzeugt, auf Basis des Teilbildes PI₂₂ wird das synthetische Teilbild PS₂₂ erzeugt, auf Basis des Teilbildes PI₂₃ wird das synthetische Teilbild PS₂₃ erzeugt, auf Basis des Teilbildes PI₂₄ wird das synthetische Teilbild PS₂₄ erzeugt, auf Basis des Teilbildes PI₂₅ wird das synthetische Teilbild PS₂₅ erzeugt und auf Basis des Teilbildes PI₂₆ wird das synthetische Teilbild PS₂₆ erzeugt.

Jedes synthetische Teilbild repräsentiert vorzugsweise denselben Teil des Untersuchungsbereichs wie das Teilbild, auf dessen Basis es erzeugt worden ist. Jedes synthetische Teilbild repräsentiert also vorzugsweise einen anderen Teilbereich des Untersuchungsbereichs.

In Fig. 2 ist ferner dargestellt, dass die synthetischen Teilbilder PS₂₁, PS₂₂, PS₂₃, PS₂₄, PS₂₅ und PS₂₆ in einem weiteren Schritt zu einem synthetischen Bild S2 zusammengefügt werden können. Das synthetische Bild S2 repräsentiert vorzugsweise den gesamten Untersuchungsbereichs (so wie das mindestens eine empfangene Bild).

Das Verfahren, das in Fig. 2 am Beispiel der Teilbilder PS₂₁, PS₂₂, PS₂₃, PS₂₄, PS₂₅ und PS₂₆ der Kopie I₂ aus Fig. 1 dargestellt ist, wird in analoger Weise auch für die Teilbilder der übrigen Kopien I₁, I₃ und I₄ durchgeführt.

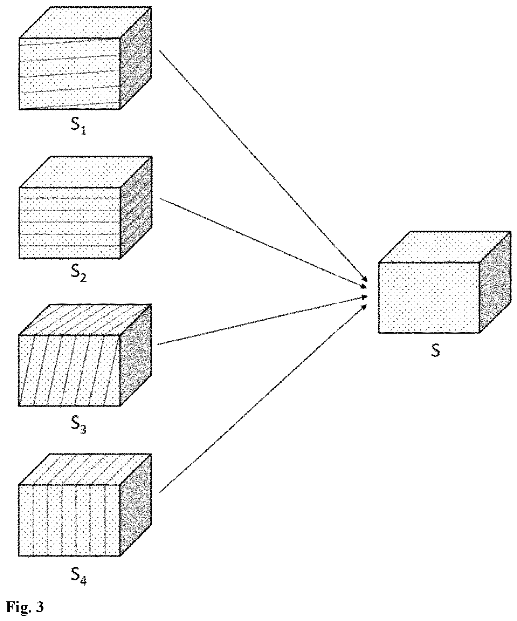

Es ergibt sich für jede Kopie I₁, I₂, I₃ und I₄ also jeweils ein synthetisches Bild S₁, S₂, S₃ und S₄. Diese sind in Fig. 3 dargestellt.

Wie in Fig. 3 schematisch dargestellt, können die synthetischen Bilder S₁, S₂, S₃ und S₄ in einem weiteren Schritt zu einem vereinten synthetischen Bild S kombiniert werden.

Die synthetischen Bilder S₁, S₂, S₃ und S₄ repräsentieren denselben Untersuchungsbereich und umfassen vorzugsweise dieselbe Zahl an Bildelementen. Jeder Teilbereich des Untersuchungsbereichs wird also durch die synthetischen Bilder S₁, S₂, S₃ und S₄ viermal repräsentiert.

Die Kombination der synthetischen Bilder S₁, S₂, S₃ und S₄ zu einem vereinten synthetischen Bild erfolgt auf Basis der Farbwerte miteinander korrespondierender Bildelemente.

Bildelemente, die denselben Teilbereich des Untersuchungsbereichs repräsentieren, werden in dieser Offenbarung als "miteinander korrespondierende Bildelemente" oder kurz "korrespondierende Bildelemente" bezeichnet. Korrespondierende Bildelemente können beispielsweise diejenigen Bildelemente sein, die dieselben Koordinaten aufweisen, wenn es sich bei dem jeweiligen synthetischen Bild um eine Rastergrafik handelt.

Für jedes k-Tupel korrespondierender Bildelemente der erzeugten synthetischen Bilder werden die Farbwerte ermittelt. Dabei gibt *k* die Zahl der miteinander korrespondierenden Bildelemente an. In dem in Fig. 3 gezeigten Beispiel korrespondieren immer vier Bildelemente der synthetischen Bilder S₁, S₂, S₃ und S₄ miteinander.

Von den Farbwerten kann ein Mittelwert (z.B. arithmetisches Mittel, geometrisches Mittel, quadratisches Mittel oder ein anderer Mittelwert) berechnet werden. Der Mittelwert des Farbwertes für ein Tupel korrespondierender Bildelemente kann als der Farbwert des entsprechenden Bildelements des vereinten synthetischen Bildes gesetzt werden.

Im Fall von mehreren Farbwerten (z.B. drei Farbwerten wie bei RGB-Farbwerten) kann für jeden Farbkanal ein Mittelwert gebildet werden. Der jeweilige Mittelwert kann dann als der Farbwert des entsprechenden Farbkanals des korrespondierenden Bildelements des vereinten synthetischen Bildes gesetzt werden.

Es ist auch denkbar, für korrespondierende Bildelemente keinen Mittelwert, sondern einen maximalen oder minimalen Farbwert zu ermitteln, und das vereinte synthetische Bild aus den Bildelementen mit den jeweiligen maximalen oder minimalen Farbwerten zusammenzusetzen. Anstelle der Maxima/Minima können auch andere statistische Größen ermittelt und zum Erzeugen des vereinten synthetischen Bildes verwendet werden.

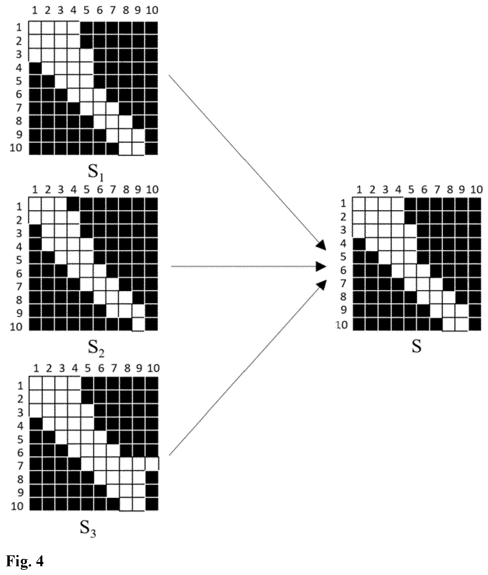

Fig. 4 zeigt beispielhaft und schematisch das Kombinieren von synthetischen Bildern zu einem vereinten synthetischen Bild.

Es sind drei synthetische Bilder S₁, S₂ und S₃ dargestellt. Bei den in Fig. 4 dargestellten synthetischen Bildern S₁, S₂ und S₃ handelt es sich um 2D-Rastergrafiken. Jedes der synthetischen Bilder S₁, S₂ und S₃ repräsentiert denselben Untersuchungsbereich eines Untersuchungsobjekts.

Jedes der drei synthetischen Bilder S₁, S₂ und S₃ weist eine Zahl von 10 - 10 = 100 Bildelementen auf. Die Bildelemente sind rasterförmig angeordnet; jeder Zeile und jeder Spalte ist eine Ziffer zugeordnet, so dass jedes Bildelement durch seine Koordinaten (Zeilenwert, Spaltenwert) eindeutig angegeben werden kann.

Bei den synthetischen Bildern S₁, S₂ und S₃ handelt es sich um Binärbilder, d.h. jedem Bildelement ist entweder der Farbwert "weiß" oder der Farbwert "schwarz" zugeordnet.

Das vereinte synthetische Bild S wird durch Kombinieren der synthetischen Bildern S₁, S₂ und S₃ erzeugt. Das Kombinieren erfolgt auf Basis von korrespondierenden Bildelementen. Korrespondierende Bildelemente repräsentieren jeweils denselben Teilbereich des Untersuchungsbereich des Untersuchungsobjekts. Im vorliegenden Beispiel stimmen die Koordinaten korrespondierender Bildelemente überein. Das Bildelement mit den Koordinaten (1,1) des synthetischen Bildes S₁ korrespondiert beispielsweise mit dem Bildelement mit den Koordinaten (1,1) des synthetischen Bildes S₂ und mit dem Bildelement mit den Koordinaten (1,1) des synthetischen Bildes S₃. Die Bildelemente mit den Koordinaten (1,1) der synthetischen Bilder S₁, S₂ und S₃ bilden ein Tupel korrespondierender Bildelemente.

Für jedes Tupel korrespondierender Bildelemente werden die Farbwerte ermittelt und es wird auf Basis der ermittelten Farbwerte der Farbwert des korrespondierenden Bildelements des vereinten synthetischen Bildes ermittelt.

Im vorliegenden Beispiel werden die synthetischen Bilder nach der folgenden Regel zu dem vereinten synthetischen Bild kombiniert: der Farbwert eines jeden Bildelements des vereinten synthetischen Bildes S entspricht dem Farbwert der Mehrheit der Farbwerte der korrespondierenden Bildelemente der synthetischen Repräsentationen S₁, S₂ und S₃.

Der Farbwert für das Bildelement mit den Koordinaten (1,1) der synthetischen Repräsentation S₁ ist beispielsweise "weiß". Der Farbwert für das korrespondierende Bildelement mit den Koordinaten (1,1) der synthetischen Repräsentation S₂ ist ebenfalls "weiß". Der Farbwert für das korrespondierende Bildelement mit den Koordinaten (1,1) der synthetischen Repräsentation S₃ ist ebenfalls "weiß". Die Mehrheit der korrespondierenden Bildelemente (nämlich alle Bildelemente) hat den Farbwert "weiß". Dementsprechend wird der Farbwert des Bildelements mit den Koordinaten (1,1) des vereinten synthetischen Bildes ebenfalls auf "weiß" gesetzt.

Der Farbwert für das Bildelement mit den Koordinaten (1,4) der synthetischen Repräsentation S₁ ist beispielsweise "weiß". Der Farbwert für das korrespondierende Bildelement mit den Koordinaten (1,4) der synthetischen Repräsentation S₂ ist "schwarz". Der Farbwert für das korrespondierende Bildelement mit den Koordinaten (1,4) der synthetischen Repräsentation S₃ ist "weiß". Die Mehrheit der korrespondierenden Bildelemente hat den Farbwert "weiß". Dementsprechend wird der Farbwert des Bildelements mit den Koordinaten (1,4) des vereinten synthetischen Bildes auf "weiß" gesetzt.

Der Farbwert für das Bildelement mit den Koordinaten (7,10) der synthetischen Repräsentation S₁ ist beispielsweise "schwarz". Der Farbwert für das korrespondierende Bildelement mit den Koordinaten (7,10) der synthetischen Repräsentation S₂ ist ebenfalls "schwarz". Der Farbwert für das korrespondierende Bildelement mit den Koordinaten (7,10) der synthetischen Repräsentation S₃ ist "weiß". Die Mehrheit der korrespondierenden Bildelemente hat den Farbwert "schwarz". Dementsprechend wird der Farbwert des Bildelements mit den Koordinaten (7,10) des vereinten synthetischen Bildes auf "schwarz" gesetzt.

Weitere Möglichkeiten, die einzelnen synthetischen Bilder zu einem vereinten synthetischen Bild zu kombinieren, sind denkbar. So kann beispielsweise ein Modell des maschinellen Lernens (z.B. ein künstliches neuronales Netzwerk) trainiert werden, das vereinte synthetische Bild nach vorgegebenen Gesichtspunkten aus den synthetischen Bildern der Mehrzahl an synthetischen Bildern zu erzeugen. Sind Trainingsdaten vorhanden, die neben synthetischen Bildern als Eingabedaten auch Bilder umfassen, die als Zieldaten verwendet werden können, kann das Modell des maschinellen Lernens in einem überwachten Lernverfahren trainiert werden, synthetische Bilder einer Mehrzahl an synthetischen Bildern zu einem vereinten synthetischen Bild zu kombinieren. Dabei können beispielsweise Aufmerksamkeitsmechanismen (engl. *attention,* siehe z.B. arXiv:2203.14263) verwendet werden, bei denen beispielsweise den einzelnen synthetischen Bildern der Mehrzahl an synthetischen Bildern unterschiedliche Gewichte bei der Kombination zu dem vereinten synthetischen Bild zugeordnet werden.

Es ist auch möglich, dass die Methode zur Erzeugung eines vereinten synthetischen Bildes nicht für jedes Tupel korrespondierender Bildelemente gleich ist. Es ist möglich, dass für verschiedene Teilbereiche des Untersuchungsobjekts verschiedene Methoden zur Erzeugung des vereinten synthetischen Bildes verwendet werden. Es ist zum Beispiel möglich, dass für Bildelemente, die ein spezifisches Gewebe und/oder ein Organ und/oder eine Läsion repräsentieren, eine andere Methode zum Kombinieren der Farbwerte der Bildelemente verwendet wird, als für Bildelemente, die ein anderes Gewebe und/oder ein anderes Organ und/oder einen anderen Bereich repräsentieren. Bereiche, für die verschiedene Methoden zum Kombinieren korrespondierender Bildelemente gelten, können beispielsweise mit Hilfe einer Segmentierung identifiziert werden.

Der Begriff "Segmentierung" bezieht sich auf den Prozess der Aufteilung eines Bildes in mehrere Segmente, die auch als Bildsegmente, Bildregionen oder Bildobjekte bezeichnet werden. Die Segmentierung wird in der Regel verwendet, um Objekte und Grenzen (Linien, Kurven usw.) in Bildern zu lokalisieren. In einem segmentierten Bild können die lokalisierten Objekte vom Hintergrund getrennt, visuell hervorgehoben (z.B. farbig), gemessen, gezählt oder anderweitig quantifiziert werden. Bei der Segmentierung wird jedem Bildelement eines Bildes ein Kennzeichen (z.B. eine Zahl) zugewiesen, so dass Bildelemente mit demselben Kennzeichen bestimmte gemeinsame Merkmale aufweisen, z.B. das gleiche Gewebe (z.B. Knochengewebe oder Fettgewebe oder gesundes Gewebe oder erkranktes Gewebe (z.B. Tumorgewebe) oder Muskelgewebe und/oder dergleichen) und/oder dasselbe Organ repräsentieren. Für korrespondierende Bildelemente mit einem spezifischen Kennzeichen kann dann eine spezifische Rechenvorschrift zum Kombinieren ihrer Farbwerte zur Erzeugung einer vereinten synthetischen Repräsentation verwendet werden; für korrespondierende Bildelemente mit einem anderen (spezifischen) Kennzeichen kann eine andere (spezifische) Rechenvorschrift z zum Kombinieren ihrer Farbwerte verwendet werden.

Es ist auch möglich, dass mehr als ein vereintes synthetisches Bild erzeugt wird, z.B. zwei oder drei oder vier oder mehr als vier. Es kann zum Beispiel ein erstes vereintes synthetisches Bild mit den jeweiligen Maxima der Farbwerte erzeugt werden und ein zweites vereintes synthetisches Bild mit den jeweiligen Minima der Farbwerte. Es kann auch ein drittes vereintes synthetisches Bild mit Mittelwerten der Farbwerte erzeugt werden.

Das vereinte synthetische Bild kann ausgegeben (z.B. auf einem Monitor angezeigt und/oder auf einem Drucker ausgegeben) und/oder in einem Datenspeicher gespeichert und/oder an ein separates Computersystem z.B. über eine Netzwerkverbindung übermittelt werden. Auf Basis des ausgegebenen vereinten synthetischen Bildes kann z.B. ein Arzt eine Diagnose stellen und/oder eine Therapie initiieren.

Es ist auch möglich, dass kein vereintes synthetisches Bild erzeugt und/oder ausgegeben wird. Es ist möglich, dass die nachfolgend beschriebene Analyse korrespondierender Bildelemente synthetischer Bilder ergibt, dass ein vereintes synthetisches Bild eine geringe Vertrauenswürdigkeit aufweist. Zum Beispiel kann ein ermittelter Vertrauenswert, der positiv mit der Vertrauenswürdigkeit korreliert, geringer als ein vordefinierter Grenzwert sein. Es kann sein, dass der ermittelte Vertrauenswert und damit die Vertrauenswürdigkeit so gering ist, dass auf Basis des vereinten synthetischen Bildes keine Diagnose erstellt und/oder Therapiemaßnahmen initiiert werden sollten. In einem solchen Fall kann es sein, dass ein vereintes synthetisches Bild wertlos oder sogar irreführend und damit gefährlich ist. Auf die Erzeugung und/oder Ausgabe eines solchen vereinten synthetischen Bildes mit einer geringen Vertrauenswürdigkeit kann dann verzichtet werden.

In dem in Fig. 1, Fig. 2 und Fig. 3 dargestellten Beispiel wird auf Basis eines einzigen empfangenen Bildes Iᵢ genau ein vereintes synthetisches Bild S erzeugt. Es ist aber möglich, auf Basis von zwei oder mehr empfangenen Bilder genau ein vereintes synthetisches Bild S zu erzeugen.

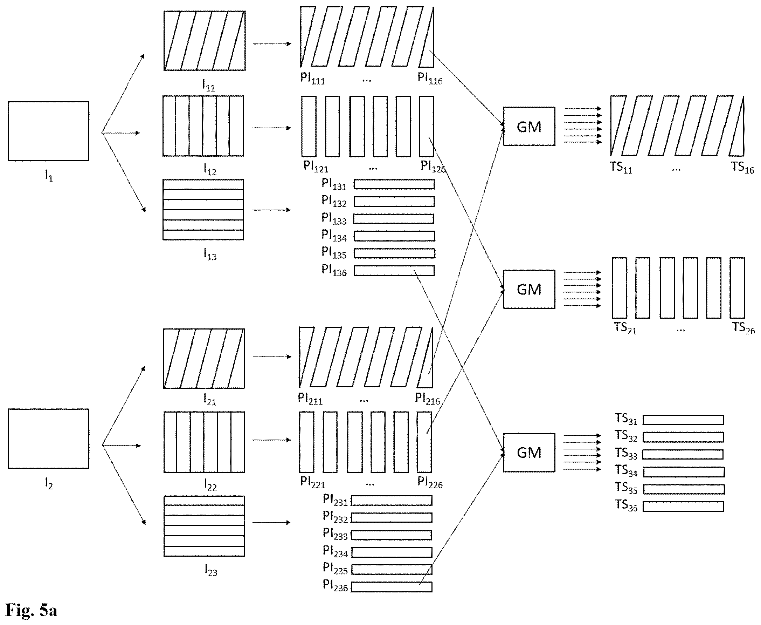

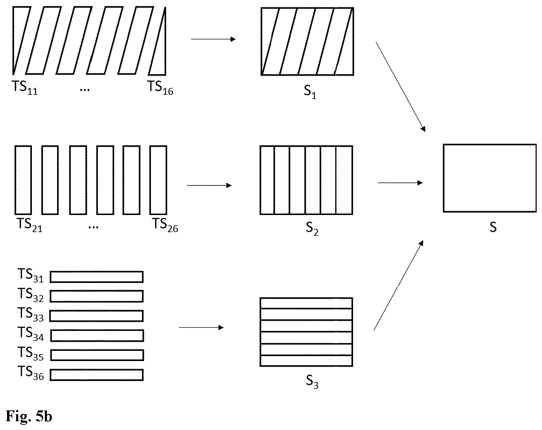

In einem solchen Fall werden von jedem empfangenen Bild Teilbilder wie in dieser Offenbarung beschrieben erzeugt. Dem generativen Modell werden dann Teilbilder, die von verschiedenen empfangenen Bildern erzeugt werden, gemeinsam zugeführt und das generative Modell erzeugt auf Basis der zugeführten Teilbilder ein synthetisches Teilbild. Die Teilbilder, die dem generativen Modell zugeführt werden, repräsentieren vorzugsweise denselben Teilbereich des Untersuchungsbereichs. Die synthetischen Teilbilder können dann zu einer Mehrzahl an synthetischen Bilder zusammengefügt werden und die Mehrzahl an synthetischen Bildern kann zu einem vereinten synthetischen Bild kombiniert werden. Dies ist in Fig. 5a und Fig. 5b an einem Beispiel mit zwei empfangenen Bilder schematisch dargestellt.

In Fig. 5a ist schematisch dargestellt, wie aus jedem Bild einer Mehrzahl an empfangenen Bildern Teilbilder erzeugt und auf Basis der erzeugten Teilbilder synthetische Teilbilder erzeugt werden. In Fig. 5b ist schematisch dargestellt, wie die synthetischen Teilbilder zu synthetischen Bildern zusammengefügt werden und wie die synthetischen Bilder zu einem vereinten synthetischen Bild kombiniert werden.

Ausgangspunkt des in Fig 5a gezeigten Verfahrens sind zwei empfangene Bilder, ein erstes Bild I₁ und ein zweites Bild I₂. Jedes Bild repräsentiert vorzugsweise denselben Untersuchungsbereich desselben Untersuchungsobjekts.

Von jedem empfangenen Bild wird eine Mehrzahl an Kopien erzeugt. In dem in Fig. 5a dargestellten Beispiel werden von jedem empfangenen Bild jeweils drei Kopien erzeugt; von dem ersten Bild I₁ werden die Kopien I₁₁, I₁₂ und I₁₃ erzeugt, von dem zweiten Bild I₂ werden die Kopien I₂₁, I₂₂ und I₂₃ erzeugt. Es ist möglich, dass eine der Kopien I₁₁, I₁₂ oder I₁₃ das erste Bild I₁ selbst ist; ebenso ist es möglich, dass eine der Kopien I₂₁, I₂₂ oder I₂₃ das zweite Bild I₂ selbst ist.

Jede Kopie wird in Teilbilder unterteilt; jedes Teilbild repräsentiert einen Teilbereich des Untersuchungsbereichs des Untersuchungsobjekts, Teilbereiche, die von verschiedenen Teilbildern repräsentiert werden, überlappen sich teilweise aber nicht vollständig. Von der Kopie I₁₁ werden die Teilbilder PI₁₁₁, PI₁₁₂, PI₁₁₃, PI₁₁₄, PI₁₁₅ und PI₁₁₆ erzeugt; von der Kopie I₁₂ werden die Teilbilder PI₁₂₁, PI₁₂₂, PI₁₂₃, PI₁₂₄, PI₁₂₅ und PI₁₂₆ erzeugt; von der Kopie I₁₃ werden die Teilbilder PI₁₃₁, PI₁₃₂, PI₁₃₃, PI₁₃₄, PI₁₃₅ und PI₁₃₆ erzeugt; von der Kopie I₂₁ werden die Teilbilder PI₂₁₁, PI₂₁₂, PI₂₁₃, PI₂₁₄, PI₂₁₅ und PI₂₁₆ erzeugt; von der Kopie I₂₂ werden die Teilbilder PI₂₂₁, PI₂₂₂, PI₂₂₃, PI₂₂₄, PI₂₂₅ und PI₂₂₆ erzeugt; von der Kopie I₂₃ werden die Teilbilder PI₂₃₁, PI₂₃₂, PI₂₃₃, PI₂₃₄, PI₂₃₅ und PI₂₃₆ erzeugt.

Miteinander korrespondierende Teilbilder, die von verschiedenen empfangenen Bildern stammen, werden zusammen einem generativen Modell GM zugefügt. "Miteinander korrespondierende Teilbilder" sind solche, die denselben Teilbereich des Untersuchungsbereichs repräsentieren.

Das generative Modell GM ist in Fig 5a zum besseren Verständnis dreimal dargestellt; es handelt sich aber stets um dasselbe generative Modell.

In dem in Fig. 5a dargestellt Beispiel korrespondiert das Teilbild PI₁₁₁ mit dem Teilbild PI₂₁₁, das Teilbild PI₁₁₂ mit dem Teilbild PI₂₁₂, das Teilbild PI₁₁₃ mit dem Teilbild PI₂₁₃, das Teilbild PI₁₁₄ mit dem Teilbild PI₂₁₄, das Teilbild PI₁₁₅ mit dem Teilbild PI₂₁₅, das Teilbild PI₁₁₆ mit dem Teilbild PI₂₁₆, das Teilbild PI₁₂₁ mit dem Teilbild PI₂₂₁, das Teilbild PI₁₂₂ mit dem Teilbild PI₂₂₂, das Teilbild PI₁₂₃ mit dem Teilbild PI₂₂₃, das Teilbild PI₁₂₄ mit dem Teilbild PI₂₂₄, das Teilbild PI₁₂₅ mit dem Teilbild PI₂₂₅, das Teilbild PI₁₂₆ mit dem Teilbild PI₂₂₆, das Teilbild PI₁₃₁ mit dem Teilbild PI₂₃₁, das Teilbild PI₁₃₂ mit dem Teilbild PI₂₃₂, das Teilbild PI₁₃₃ mit dem Teilbild PI₂₃₃, das Teilbild PI₁₃₄ mit dem Teilbild PI₂₃₄, das Teilbild PI₁₃₅ mit dem Teilbild PI₂₃₅ und das Teilbild PI₁₃₆ mit dem Teilbild PI₂₃₆.

Dem generativen Modell GM werden die jeweils miteinander korrespondierenden Teilbilder zusammen zugeführt und das generative Modell erzeugt auf Basis der zugeführten Teilbilder jeweils ein synthetisches Teilbild. Das jeweilige synthetische Teilbild korrespondiert mit den Teilbilder, auf deren Basis es erzeugt wurde, d.h., es repräsentiert denselben Teilbereich des Untersuchungsbereichs wie die Teilbilder auf deren Basis es erzeugt wurde.

In dem in Fig. 5a dargestellten Beispiel erzeugt das generative Modell GM auf Basis der Teilbilder PI₁₁₁ und PI₂₁₁, das synthetische Teilbild TS₁₁, auf Basis der Teilbilder PI₁₁₂ und PI₂₁₂ das synthetische Teilbild TS₁₂, auf Basis der Teilbilder PI₁₁₃ und PI₂₁₃ das synthetische Teilbild TS₁₃, auf Basis der Teilbilder PI₁₁₄ und PI₂₁₄ das synthetische Teilbild TS₁₄, auf Basis der Teilbilder PI₁₁₅ und PI₂₁₅ das synthetische Teilbild TS₁₅, auf Basis der Teilbilder PI₁₁₆ und PI₂₁₆ das synthetische Teilbild TS₁₆, auf Basis der Teilbilder PI₁₂₁ und PI₂₂₁ das synthetische Teilbild TS₂₁, auf Basis der Teilbilder PI₁₂₂ und PI₂₂₂ das synthetische Teilbild TS₂₂, auf Basis der Teilbilder PI₁₂₃ und PI₂₂₃ das synthetische Teilbild TS₂₃, auf Basis der Teilbilder PI₁₂₄ und PI₂₂₄ das synthetische Teilbild TS₂₄, auf Basis der Teilbilder PI₁₂₅ und PI₂₂₅ das synthetische Teilbild TS₂₅, auf Basis der Teilbilder PI₁₂₆ und PI₂₂₆ das synthetische Teilbild TS₂₆, auf Basis der Teilbilder PI₁₃₁ und PI₂₃₁ das synthetische Teilbild TS₃₁, auf Basis der Teilbilder PI₁₃₂ und PI₂₃₂ das synthetische Teilbild TS₃₂, auf Basis der Teilbilder PI₁₃₃ und PI₂₃₃ das synthetische Teilbild TS₃₃, auf Basis der Teilbilder PI₁₃₄ und PI₂₃₄ das synthetische Teilbild TS₃₄, auf Basis der Teilbilder PI₁₃₅ und PI₂₃₅ das synthetische Teilbild TS₃₅ und auf Basis der Teilbilder PI₁₃₆ und PI₂₃₆ das synthetische Teilbild TS₃₆.

Die synthetischen Teilbilder, die auf Basis derselben Unterteilungen der Kopien erzeugt wurden, werden in einem nächsten Schritt zu synthetischen Bildern zusammengefügt.

In dem in Fig. 5b gezeigten Beispiel werden die Teilbilder TS₁₁, TS₁₂, TS₁₃, TS₁₄, TS₁₅ und TS₁₆ zu dem synthetischen Bild S₁ zusammengefügt; die Teilbilder TS₂₁, TS₂₂, TS₂₃, TS₂₄, TS₂₅ und TS₂₆ werden zu dem synthetischen Bild S₂ zusammengefügt und die Teilbilder TS₃₁, TS₃₂, TS₃₃, TS₃₄, TS₃₅ und TS₃₆ werden zu dem synthetischen Bild S₃ zusammengefügt.

Die synthetischen Bilder S₁, S₂ und S₃ werden in einem weiteren Schritt zu einem vereinten synthetischen Bild S kombiniert. Dieser Vorgang entspricht dem in Fig. 3 und Fig. 4 dargestellten Vorgang. Das vereinte synthetische Bild S kann ausgegeben (z.B. auf einem Monitor angezeigt und/oder auf einem Drucker ausgegeben) und/oder in einem Datenspeicher gespeichert und/oder an ein separates Computersystem z.B. über eine Netzwerkverbindung übertragen werden.

Im Folgenden wird die Ermittlung des mindestens einen Vertrauenswertes näher beschrieben.

Der mindestens eine Vertrauenswert kann ein Wert sein, der angibt, inwieweit man einem synthetischen Bild (z.B. dem vereinten synthetischen Bild) vertrauen kann. Der Vertrauenswert kann positiv mit der Vertrauenswürdigkeit des synthetischen Bildes korrelieren, d.h., wenn der Vertrauenswert gering ist, ist auch die Vertrauenswürdigkeit gering und wenn der Vertrauenswert hoch ist, ist auch die Vertrauenswürdigkeit hoch. Es ist aber auch möglich, dass der Vertrauenswert negativ mit der Vertrauenswürdigkeit korreliert; d.h., wenn der Vertrauenswert gering ist, ist die Vertrauenswürdigkeit hoch und wenn der Vertrauenswert hoch ist, ist die Vertrauenswürdigkeit gering. Bei einer negativen Korrelation kann man anstelle des Vertrauenswerts auch von einem Unsicherheitswert sprechen: ist der Unsicherheitswert hoch, dann geht von dem synthetischen Bild eine hohe Unsicherheit aus; es ist möglich, dass das synthetische Bild ein oder mehrere Artefakte aufweist; es ist möglich, dass für Strukturen und/oder Morphologien und/oder Texturen in dem synthetischen Bild keine Entsprechung in der Realität existiert, d.h., dass Strukturen und/oder Morphologien und/oder Texturen in dem synthetischen Bild nicht auf reale Strukturen und/oder reale Morphologien und/oder reale Texturen im Untersuchungsbereich zurückgeführt werden können. Ein geringer Unsicherheitswert hingegen zeigt an, dass das synthetische Bild eine geringe Unsicherheit aufweist; Merkmale in dem synthetischen Bild haben eine Entsprechung in der Realität; dem synthetischen Bild kann vertraut werden; eine medizinische Diagnose kann auf Basis des synthetischen Bildes gestellt und/oder eine medizinische Therapie kann auf Basis des synthetischen Bildes initiiert werden.

Ein Vertrauenswert, der positiv mit der Vertrauenswürdigkeit korreliert, kann prinzipiell auch in einen Vertrauenswert, der negativ mit der Vertrauenswürdigkeit korreliert (einen Unsicherheitswert), umgewandelt werden, beispielsweise, indem man das Reziproke (den Kehrwert) bildet. Umgekehrt kann entsprechend auch ein Vertrauenswert, der negativ mit der Vertrauenswürdigkeit korreliert (ein Unsicherheitswert), in einen Vertrauenswert, der positiv mit der Vertrauenswürdigkeit korreliert, umgewandelt werden.

Der mindestens eine Vertrauenswert kann auf Basis korrespondierender Bildelemente synthetischer Repräsentationen ermittelt werden.

Für jedes *k*-Tupel korrespondierender Bildelemente der erzeugten synthetischen Bilder werden die Farbwerte ermittelt. Dabei gibt *k* die Zahl der miteinander korrespondierenden Bildelemente an. In dem in Fig. 4 dargestellten Beispiel korrespondieren jeweils drei Bildelemente der synthetischen Bilder S₁, S₂ und S₃ miteinander.

Je mehr sich die Farbwerte korrespondierender Bildelemente in den synthetischen Bildern unterscheiden, desto einen größeren Unterschied macht es, auf welchen Teilbildern die Erzeugung eines synthetischen Bildes basiert. Wenn es jedoch einen großen Unterschied macht, auf welchen Teilbildern ein synthetisches Bild basiert, dann geht von dem synthetischen Bild eine gewisse Unsicherheit aus; die Vertrauenswürdigkeit ist umso geringer, je größer die Unterschiede sind.

Daher kann das Ausmaß, in dem sich Farbwerte korrespondierender Bildelemente unterscheiden, als Maß für die Vertrauenswürdigkeit/Unsicherheit verwendet werden: je mehr sich Farbwerte korrespondierender Bildelemente unterscheiden, desto geringer ist die Vertrauenswürdigkeit, desto höher ist die Unsicherheit; je weniger sich Farbwerte korrespondierender Bildelemente unterscheiden, desto geringer ist die Unsicherheit, desto höher ist die Vertrauenswürdigkeit.

Die Vertrauenswürdigkeit/Unsicherheit kann also für jedes Tupel korrespondierender Bildelemente synthetischer Bilder der Mehrzahl an synthetischen Bildern ermittelt werden und repräsentiert dann die Vertrauenswürdigkeit/Unsicherheit (i) jedes synthetischen Bildes der Mehrzahl an synthetischen Bilder, (ii) der Gesamtheit der synthetischen Bilder der Mehrzahl an synthetischen Bildern und (iii) des vereinten synthetischen Bildes.

Mit anderen Worten: es lässt sich für jedes einzelne Bildelement des vereinten synthetischen Bildes ein Vertrauenswert ermitteln, der angibt, wie sehr man dem Farbwert des Bildelements vertrauen kann.

Ein solcher Vertrauenswert kann beispielsweise die Streubreite des Tupels korrespondierender Bildelemente sein. Die Streubreite ist definiert als die Differenz zwischen dem größten Wert und dem kleinsten Wert einer Variable. Es kann also für jedes Tupel korrespondierender Bildelemente ein maximaler Farbwert und ein minimaler Farbwert ermittelt und die Differenz zwischen dem maximalen und dem minimalen Farbwert gebildet werden. Das Ergebnis ist die Streubreite der Farbwerte des Tupels korrespondierender Bildelemente, die als Vertrauenswert verwendet werden kann.

Gibt es mehr als einen Farbwert (zum Beispiel drei Farbwerte, wie im Fall von Bildern, deren Farbwerte gemäß dem RGB-Farbmodell spezifiziert sind), kann für jeden Farbkanal ein maximaler und ein minimaler Farbwert ermittelt und die Differenz für jeden Farbkanal gebildet werden. Es ergeben sich drei Streubreiten. Es kann für jeden Farbkanal die jeweilige Streubreite als ein eigener Vertrauenswert verwendet werden; es ist aber auch möglich, die Streubreiten der Farbkanäle zu einem einzigen Wert zu kombinieren; es ist möglich, die maximale Streubreite als Vertrauenswert zu verwenden; es ist möglich einen Mittelwert (z.B. das arithmetische Mittel, das geometrische Mittel, das quadratische Mittel oder einen anderen Mittelwert) der Streubreiten als Vertrauenswert zu verwenden; es ist möglich, die Länge des Vektors, den die Streubreiten in einem dreidimensionalen Raum (oder einem höherdimensionalen Raum bei Verwendung von mehr als drei Farbkanälen) spezifizieren, als Vertrauenswert zu verwenden; weitere Möglichkeiten sind denkbar.

Ein Vertrauenswert für ein Tupel korrespondierender Bildelemente kann auch die Varianz und/oder die Standardabweichung der Farbwerte der korrespondierenden Bildelemente sein. Die Varianz ist definiert als die mittlere quadratische Abweichung einer Variablen von ihrem Erwartungswert; die Standardabweichung ist definiert als die Quadratwurzel der Varianz.

Ein Vertrauenswert kann auch ein anderes Streuungsmaß sein, wie beispielweise die Summe der Abweichungsquadrate, der Variationskoeffizient, die mittlere absolute Abweichung, ein Quantilsabstand, ein Interquantilsabstand, der mittlere absolute Abstand vom Median, der Median der absoluten Abweichungen und/oder die geometrische Standardabweichung. Es ist auch möglich, dass es mehr als einen Vertrauenswert für ein Tupel korrespondierender Bildelemente gibt.

Es ist auch möglich, dass die Methode zur Berechnung eines Vertrauenswerts nicht für jedes Tupel korrespondierender Bildelemente gleich ist. Es ist möglich, dass für verschiedene Teilbereiche des Untersuchungsobjekts verschiedene Methoden zur Berechnung eines Vertrauenswertes verwendet werden. Es ist zum Beispiel möglich, dass für Bildelemente, die ein spezifisches Gewebe und/oder ein Organ und/oder eine Läsion repräsentieren, eine andere Methode zur Berechnung eines Vertrauenswerts verwendet wird, als für Bildelemente, die ein anderes Gewebe und/oder ein anderes Organ und/oder einen anderen Bereich repräsentieren. Bereiche, für die verschiedene Rechenvorschriften für die Vertrauenswerte gelten, können beispielsweise mit Hilfe einer Segmentierung identifiziert werden.

Bei der Segmentierung kann jedem Bildelement eines Bildes ein Kennzeichen (z.B. eine Zahl) zugewiesen werden, so dass Bildelemente mit demselben Kennzeichen bestimmte gemeinsame Merkmale aufweisen, z.B. das gleiche Gewebe (z.B. Knochengewebe oder Fettgewebe oder gesundes Gewebe oder erkranktes Gewebe (z.B. Tumorgewebe) oder Muskelgewebe und/oder dergleichen) und/oder dasselbe Organ repräsentieren. Für korrespondierende Bildelemente mit einem spezifischen Kennzeichen kann dann eine spezifische Rechenvorschrift zur Berechnung eines Vertrauenswerts verwendet werden; für korrespondierende Bildelemente mit einem anderen (spezifischen) Kennzeichen kann eine andere (spezifische) Rechenvorschrift zur Berechnung eines Vertrauenswerts verwendet werden.

Die für Tupel korrespondierender Bildelemente ermittelten Vertrauenswerte können ausgegeben (z.B. auf einem Monitor angezeigt oder auf einem Drucker ausgedruckt), in einem Datenspeicher gespeichert und/oder an ein separates Computersystem z.B. über ein Netzwerk übermittelt werden.

Die für Tupel korrespondierender Bildelemente ermittelten Vertrauenswerte können auch bildhaft dargestellt werden.

Neben dem vereinten synthetischen Bild kann also eine weitere Repräsentation des Untersuchungsbereichs ausgegeben (z.B. auf einem Monitor angezeigt) werden, in der für jedes Bildelement angezeigt wird, wie vertrauenswürdig es ist. Eine solche Repräsentation wird in dieser Beschreibung auch als Vertrauensrepräsentation bezeichnet. Die Vertrauensrepräsentation hat vorzugsweise die gleiche Dimension und Größe wie das vereinte synthetische Bild; jedem Bildelement des vereinten synthetischen Bildes ist vorzugsweise ein Bildelement in der Vertrauensrepräsentation zugeordnet.

Anhand einer solchen Vertrauensrepräsentation kann ein Nutzer (z.B. ein Arzt) für jedes einzelne Bildelement erkennen, wie weit er dem Farbwert des Bildelements vertrauen kann. Es ist möglich, die Vertrauensrepräsentation mit dem vereinten synthetischen Bild und/oder mit einem empfangenen Bild ganz oder teilweise überlagert darzustellen. Es ist möglich, die überlagerte Darstellung so zu gestalten, dass der Nutzer sie ein- und ausblenden kann. Der Nutzer kann sich das vereinte synthetische Bild und/oder ein empfangenes Bild beispielsweise Schicht für Schicht anzeigen lassen, so wie es für computertomografische-, magnetresonanztomografische und andere drei- oder höherdimensionale Repräsentationen üblich ist. Bei jeder Schicht kann er die entsprechende Schicht der Vertrauensrepräsentation einblenden lassen, um zu prüfen, ob Bildelemente in der Schicht, die Strukturen, Morphologien und/oder Texturen in der vereinten synthetischen Repräsentation zeigen, vertrauenswürdig oder unsicher sind. Auf diese Weise kann der Nutzer erkennen, wie hoch das Risiko ist, dass es sich bei den Strukturen, Morphologien und/oder Texturen um reale Eigenschaften des Untersuchungsbereichs oder um Artefakte handelt.

Bildelemente mit einer geringen Vertrauenswürdigkeit (mit einer hohen Unsicherheit) können beispielsweise hell und/oder mit einer Signalfarbe (z.B. rot oder orange oder gelb) dargestellt werden, während Bildelemente mit einer hohen Vertrauenswürdigkeit (mit einer geringen Unsicherheit) dunkel oder mit einer unauffälligeren oder beruhigenden Farbe (z.B. grün oder blau) dargestellt werden können. Es ist auch möglich, dass nur diejenigen Bildelemente bei einer Überlagerung dargestellt werden, bei denen der Vertrauenswert einen vordefinierten Grenzwert über- oder unterschreitet. Korreliert der Vertrauenswert positiv mit der Vertrauenswürdigkeit können beispielsweise nur diejenigen Bildelemente der Vertrauensrepräsentation dargestellt werden, deren Vertrauenswert unterhalb eines vordefinierten Grenzwerts liegt; in einem solchen Fall werden einem Nutzer (z.B. einem Arzt) nur diejenigen Bildelemente zur Kenntnis gegeben, denen er eher nicht vertrauen sollte.

Es ist auch möglich, dass Vertrauenswerte für Teilbereiche (Teilbilder) des vereinten synthetischen Bildes (z.B. Schichten innerhalb des vereinten synthetischen Bildes) und/oder für das gesamte vereinte synthetische Bild ermittelt werden. Eine Ermittlung solcher Vertrauenswerte für Teilbereiche oder gesamte Bilder kann auf Basis der Vertrauenswerte derjenigen Bildelemente erfolgen, aus denen sie zusammengesetzt sind. Zur Ermittlung eines Vertrauenswerts einer Schicht können beispielsweise alle Vertrauenswerte derjenigen Bildelemente berücksichtigt werden, die in dieser Schicht liegen. Es ist aber auch möglich, auch benachbarte Bildelemente (zum Beispiel Bildelemente der Schicht über und/oder unter betrachteten Schicht) zu berücksichtigen. Ein Vertrauenswert für einen Teilbereich oder den gesamten Bereich kann beispielsweise durch Mittelwertbildung (z.B. arithmetischer Mittelwert, geometrisches Mittel, quadratische Mittel oder einen anderen Mittelwert) ermittelt werden. Es ist auch möglich, den Maximalwert (z.B. bei einem Vertrauenswert, der negativ mit der Vertrauenswürdigkeit korreliert) oder den Minimalwert (z.B. bei einem Vertrauenswert, der negativ mit der Vertrauenswürdigkeit korreliert) der Vertrauenswerte der Bildelemente eines Teilbereichs oder des Gesamtbereichs zu ermitteln und diesen als Vertrauenswert des Teilbereichs oder des Gesamtbereichs zu verwenden. Weitere Möglichkeiten, einen Vertrauenswert für einen Teilbereich oder den Gesamtbereich auf Basis der Vertrauenswerte einzelner Bildelemente zu ermitteln, sind denkbar.

Eine solcher Vertrauenswert für einen Teilbereich oder den Gesamtbereich kann ebenfalls ausgegeben (z.B. auf einem Monitor angezeigt oder auf einem Drucker ausgegeben), in einem Datenspeicher gespeichert und/oder an ein separates Computersystem übermittelt werden. Er kann auch, wie für die einzelnen Vertrauenswerte beschrieben, bildhaft (z.B. farblich) dargestellt werden.

Ist ein positiv mit der Vertrauenswürdigkeit korrelierender Vertrauenswert für einen Teilbereich oder den Gesamtbereich geringer als ein vordefinierter Grenzwert, dann ist es möglich, dass dem entsprechenden Teilbereich oder Gesamtbereich kein Vertrauen geschenkt werden sollte. Es ist möglich, dass, ein solcher Teilbereich oder der entsprechende Gesamtbereich wie oben beschrieben gar nicht ausgegeben (z.B. gar nicht angezeigt) wird, oder dass er mit einem Warnhinweis angezeigt wird, dass ein Nutzer bei der Interpretation der angezeigten Daten vorsichtig sein sollte, da die angezeigten Daten unsicher sind.

Es ist auch möglich, dass dem Nutzer des Computersystems/Computerprogramms der vorliegenden Offenbarung über eine Nutzerschnittstelle die Möglichkeit gegeben wird, in dem vereinten synthetischen Bild zu Teilbereichen zu navigieren, die eine niedrige Vertrauenswürdigkeit aufweisen. Dem Nutzer können beispielsweise die Teilbereiche mit der niedrigsten Vertrauenswürdigkeit in einer Liste angezeigt werden (z.B. in Form einer Liste mit einer Zahl *p* an Teilbereichen, die den niedrigsten positiv mit der Vertrauenswürdigkeit korrelierenden Vertrauenswert aufweisen, wobei p eine positive ganze Zahl ist). Durch ein Anklicken eines Listeneintrags kann dem Nutzer der entsprechende Teilbereich in Form eines vereinten synthetischen Bildes, einer Vertrauenskarte und/oder einem empfangenen Bild und/oder einem Ausschnitt davon angezeigt werden.

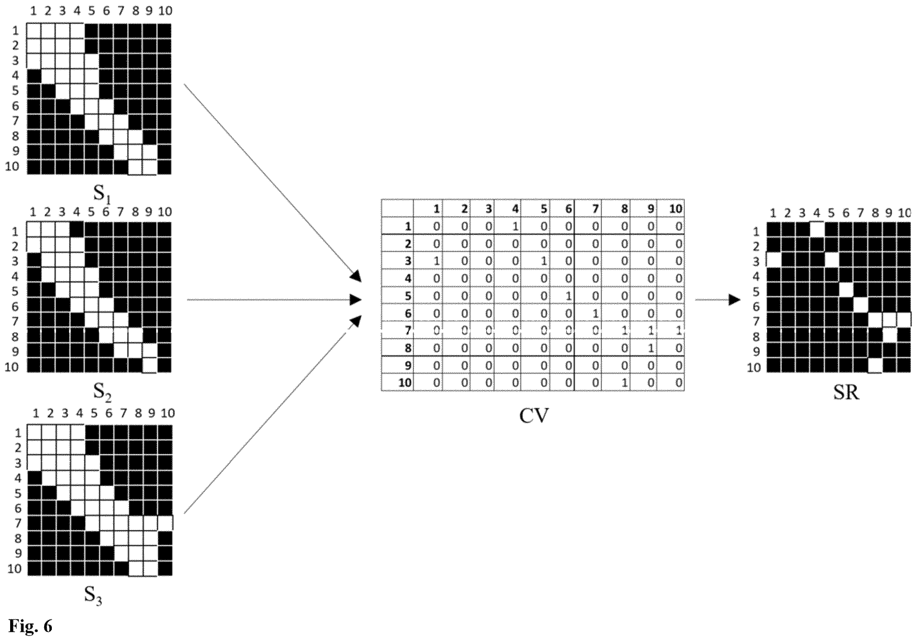

Fig. 6 zeigt beispielhaft und schematisch das Ermitteln des mindestens einen Vertrauenswertes. Das Ermitteln des mindestens einen Vertrauenswerts erfolgt auf Basis korrespondierender Bildelemente der bereits in Fig. 4 gezeigten synthetischen Bilder S₁, S₂ und S₃.

Es wird für jedes Tupel korrespondierender Bildelemente jeweils ein Vertrauenswert ermittelt. In einem ersten Schritt werden die Farbwerte aller Bildelemente ermittelt. Im vorliegenden Beispiel ist der Farbe "schwarz" wie allgemein üblich der Farbwert "0" und der Farbe "weiß" der Farbwert "1" zugeordnet.

Als Vertrauenswert wird für jedes Tupel korrespondierender Bildelemente der synthetischen Bilder S₁, S₂ und S₃ die Streubreite berechnet.

Der Farbwert für das Bildelement mit den Koordinaten (1,1) der synthetischen Repräsentation S₁ ist beispielsweise "1" (weiß). Der Farbwert für das korrespondierende Bildelement mit den Koordinaten (1,1) der synthetischen Repräsentation S₂ ist ebenfalls "1" (weiß). Der Farbwert für das korrespondierende Bildelement mit den Koordinaten (1,1) der synthetischen Repräsentation S₃ ist ebenfalls "1" (weiß). Die Streubreite für das Tupel korrespondierender Bildelemente ist also 1 - 1 = 0.

Der Farbwert für das Bildelement mit den Koordinaten (1,4) der synthetischen Repräsentation S₁ ist beispielsweise "1" (weiß). Der Farbwert für das korrespondierende Bildelement mit den Koordinaten (1,4) der synthetischen Repräsentation S₂ ist "0" (schwarz). Der Farbwert für das korrespondierende Bildelement mit den Koordinaten (1,4) der synthetischen Repräsentation S₃ ist "1" (weiß). Die Streubreite für das Tupel korrespondierender Bildelemente ist also 1 - 0 = 1.

Der Farbwert für das Bildelement mit den Koordinaten (7,10) der synthetischen Repräsentation S₁ ist beispielsweise "0" (schwarz). Der Farbwert für das korrespondierende Bildelement mit den Koordinaten (7,10) der synthetischen Repräsentation S₂ ist ebenfalls "0" (schwarz). Der Farbwert für das korrespondierende Bildelement mit den Koordinaten (7,10) der synthetischen Repräsentation S₃ ist "1" (weiß). Die Streubreite für das Tupel korrespondierender Bildelemente ist also 1 - 0 = 1.

Die Vertrauenswerte sind in der Tabelle CV aufgelistet.

Die so ermittelten Vertrauenswerte korrelieren negativ mit der Vertrauenswürdigkeit.

Auf Basis der Vertrauenswerte lässt sich eine Vertrauensrepräsentation ermitteln. In dem in Fig. 6 dargestellten Beispiel wird der Farbwert jedes Bildelements in der Vertrauensrepräsentation SR auf den entsprechenden Vertrauenswert des Tupels korrespondierender Bildelemente gesetzt. So erhält beispielsweise das Bildelement mit den Koordinaten (1,1) in der Vertrauensrepräsentation die Farbe schwarz, während die Bildelemente mit den Koordinaten (1,4) und (7,10) die Farbe weiß erhalten. Anhand der Vertrauensrepräsentation SR kann ein Nutzer (z.B. ein Arzt) sofort erkennen, welche Bildelemente sicher (schwarz) und welche unsicher (weiß) sind. Bereichen, in denen viele weiße Bildelemente in der Vertrauensrepräsentation SR auftreten, sollte der Nutzer weniger vertrauen.

Fig. 7 zeigt eine Ausführungsform des Verfahrens der vorliegenden Offenbarung in Form eines Ablaufschemas.

Das Verfahren (100) umfasst die Schritte:
- (110): Empfangen mindestens eines Bildes eines Untersuchungsbereichs eines Untersuchungsobjekts,
- (120): Erzeugen einer Vielzahl an Teilbildern auf Basis des mindestens einen empfangenen Bildes, wobei jedes Teilbild einen Teilbereich des Untersuchungsbereichs des Untersuchungsobjekts repräsentiert, wobei sich Teilbereiche, die von verschiedenen Teilbildern repräsentiert werden, teilweise aber nicht vollständig überlappen,
- (130): für jedes erzeugte Teilbild: Erzeugen eines synthetischen Teilbildes zumindest anteilig auf Basis des erzeugten Teilbildes,
- (140): Ermitteln von Farbwerten korrespondierender Bildelemente synthetischer Teilbilder, wobei korrespondierende Bildelemente denselben Teilbereich des Untersuchungsbereichs repräsentieren,
- (150): Ermitteln eines Streuungsmaßes der Farbwerte korrespondierender Bildelemente,
- (160): Ermitteln eines Vertrauenswertes auf Basis des Streuungsmaßes,
- (170): Ausgeben des Vertrauenswertes.

Wie beschrieben kann das in dieser Beschreibung beschriebene generative Modell ein trainiertes Modell des maschinellen Lernens sein. Fig. 8 zeigt beispielhaft und schematisch ein Verfahren zum Trainieren eines solchen Modell des maschinellen Lernens.

Das Trainieren des generativen Modells GM erfolgt mit Trainingsdaten TD. Die Trainingsdaten TD umfassen, für jedes Referenzobjekt einer Vielzahl an Referenzobjekten, (i) mindestens ein Referenzbild des Referenzbereichs des Referenzobjekts in mindestens einem ersten Zustand als Eingabedaten und ein Referenzbild des Referenzobjekts in mindestens einem, vom ersten Zustand abweichenden Zustand. Der Begriff "Vielzahl an Referenzobjekten" bedeutet vorzugsweise mehr als 10, noch mehr bevorzugt mehr als 100 Referenzobjekte.

Der Begriff "Referenz" wird hier verwendet, um die Trainingsphase von der Phase der Nutzung des trainierten Modells zum Erzeugen synthetischer Bilder zu unterscheiden.

Ein "Referenzbild" ist ein Bild, das zum Trainieren des Modells verwendet wird. Das "Referenzobjekt" ist ein Objekt, von dem das Referenzbild stammt. Das Referenzobjekt ist üblicherweise wie das Untersuchungsobjekt ein Tier oder ein Mensch, vorzugsweise ein Mensch. Der Referenzbereich ist ein Teil des Referenzobjekts. Vorzugsweise handelt es sich bei dem Referenzbereich um den gleichen Teil wie der Untersuchungsbereich des Untersuchungsobjekts.

Der Begriff "Referenz" hat ansonsten aber keine einschränkende Bedeutung. Aussagen, die in dieser Beschreibung zu dem mindestens einen empfangenen Bild getroffen werden, treffen in analoger Weise auf jedes Referenzbild zu; Aussagen, die in dieser Beschreibung zu dem Untersuchungsobjekt getroffen werden, treffen in analoger Weise auf jedes Referenzobjekt zu; Aussagen, die in dieser Beschreibung zu dem Untersuchungsbereich getroffen werden, treffen in analoger Weise auf den Referenzbereich zu.

In dem in Fig. 8 dargestellten Beispiel ist nur ein Satz an Trainingsdaten TD eines Referenzobjekts gezeigt; üblicherweise umfassen die Trainingsdaten TD eine Vielzahl dieser Datensätze für eine Vielzahl an Referenzobjekten. In dem in Fig. 8 dargestellten Beispiel umfassen die Trainingsdaten TD ein erstes Referenzbild RI₁, ein zweites Referenzbild RI₂ und ein drittes Referenzbild RI₃.

Das erste Referenzbild RI₁ repräsentiert den Referenzbereich des Referenzobjekts in einem ersten Zustand; das zweite Referenzbild RI₂ repräsentiert den Referenzbereich des Referenzobjekts in einem zweiten Zustand; das dritte Referenzbild RI₃ repräsentiert den Referenzbereich des Referenzobjekts in einem dritten Zustand. Der erste Zustand, der zweite Zustand und der dritte Zustand unterscheiden sich üblicherweise voneinander. Zum Beispiel kann der Zustand eine Menge an Kontrastmittel repräsentieren, die in den Referenzbereich eingebracht wird oder worden ist. Zum Beispiel kann der Zustand einen Zeitpunkt vor und/oder nach einer Applikation eines Kontrastmittels repräsentieren.

Zum Beispiel kann das erste Referenzbild RI₁ den Referenzbereich ohne oder nach Applikation einer ersten Menge eines Kontrastmittel, das zweite Referenzbild RI₂ den Referenzbereich nach Applikation einer zweiten Menge des Kontrastmittels und das dritte Referenzbild RI₃ den Referenzbereich nach Applikation einer dritten Menge des Kontrastmittels repräsentieren. Dabei kann die erste Menge kleiner als die zweite Menge und die zweite Menge kleiner als die dritte Menge sein (siehe z.B. WO2019/074938A1, WO2022184297A1).

Zum Beispiel kann das erste Referenzbild RI₁ den Referenzbereich vor oder in einer ersten Zeitspanne nach Applikation eines Kontrastmittels, das zweite Referenzbild RI₂ den Referenzbereich in einer zweiten Zeitspanne nach Applikation des Kontrastmittels und das dritte Referenzbild RI₃ den Referenzbereich in einer dritten Zeitspanne nach Applikation des Kontrastmittels repräsentieren (siehe z.B. WO2021052896A1, WO2021069338A1).

Das erste Referenzbild RI₁ und das zweite Referenzbild RI₂ dienen in dem in Fig. 8 gezeigten Beispiel als Eingabedaten; sie werden dem generativen Modell GM zugeführt. Das generative Modell GM ist konfiguriert, auf Basis des ersten Referenzbildes RI₁ und des zweiten Referenzbildes RI₂ und auf Basis von Modellparametern MP ein synthetisches Bild S zu erzeugen. Das synthetische Bild S soll dem dritten Referenzbild RI₃ möglichst nahe kommen. Das heißt, das dritte Referenzbild RI₃ fungiert in dem in Fig. 8 gezeigten Beispiel als Zieldaten *(ground truth).*

Das von dem generativen Modell GM erzeugte synthetische Bild S wird mit dem dritten Referenzbild RI₃ verglichen. Eine Fehlerfunktion LF wird verwendet, um Abweichungen zwischen dem synthetischen Bild S und dem dritten Referenzbild RI₃ zu quantifizieren. Für jedes Paar aus einem synthetischen Bild und einem dritten Referenzbild kann mittels der Fehlerfunktion LF ein Fehlerwert berechnet werden.

In einem Optimierungsverfahren können der Fehlerwert und damit die Abweichungen zwischen dem vom generativen Modell erzeugten synthetischen Bild S und dem dritten Referenzbild RI₃ durch Modifizieren von Modellparametern MP reduziert werden.

Der Prozess wird für eine Vielzahl an Referenzobjekten wiederholt.

Erreichen die Fehlerwerte ein vordefiniertes Minimum oder lassen sich die Fehlerwerte durch Modifizieren von Modellparametern nicht weiter reduzieren, kann das Training beendet werden. Das trainierte Modell kann gespeichert, an ein separates Computersystem übermittelt und/oder zur Erzeugung von synthetischen Bildern für (neue) Objekte (Untersuchungsobjekte) verwendet werden.

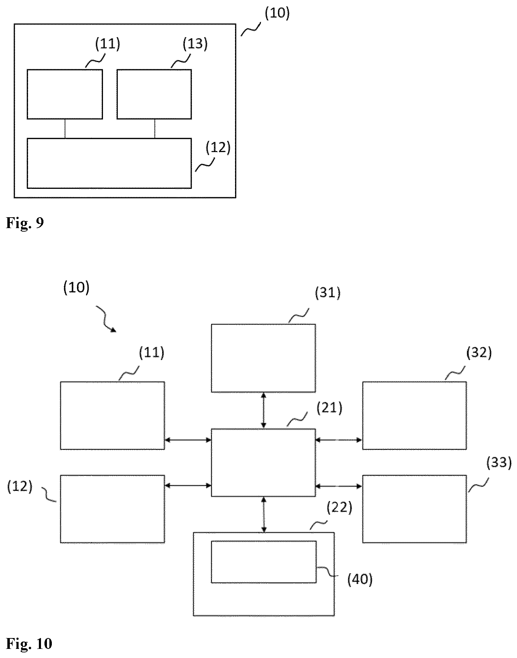

Fig. 9 zeigt beispielhaft und schematisch ein Computersystem gemäß der vorliegenden Offenbarung.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 9 gezeigte Computersystem (10) umfasst eine Empfangseinheit (11), eine Steuer- und Recheneinheit (12) und eine Ausgabeeinheit (13).

Die Steuer- und Recheneinheit (12) dient der Steuerung des Computersystems (10), der Koordinierung der Datenflüsse zwischen den Einheiten des Computersystems (10) und der Durchführung von Berechnungen.

Die Steuer- und Recheneinheit (12) ist konfiguriert:
- die Empfangseinheit (11) zu veranlassen, mindestens ein Bild eines Untersuchungsbereichs eines Untersuchungsobjekts zu empfangen,
- eine Vielzahl an Teilbildern auf Basis des mindestens einen empfangenen Bildes zu erzeugen, wobei jedes Teilbild einen Teilbereich des Untersuchungsbereichs des Untersuchungsobjekts repräsentiert, wobei sich Teilbereiche, die von verschiedenen Teilbildern repräsentiert werden, teilweise aber nicht vollständig überlappen.
- für jedes erzeugte Teilbild ein synthetisches Teilbild zumindest anteilig auf Basis des erzeugten Teilbildes zu erzeugen,
- Farbwerte korrespondierender Bildelemente synthetischer Teilbilder zu ermitteln, wobei korrespondierende Bildelemente denselben Teilbereich des Untersuchungsbereichs repräsentieren,
- ein Streuungsmaß der Farbwerte korrespondierender Bildelemente zu ermitteln,
- einen Vertrauenswert auf Basis des Streuungsmaßes zu ermitteln,
- die Ausgabeeinheit zu veranlassen, den Vertrauenswert auszugeben.

Fig. 10 zeigt beispielhaft und schematisch eine weitere Ausführungsform des Computersystems. Das Computersystem (10) umfasst eine Verarbeitungseinheit (21), die mit einem Speicher (22) verbunden ist. Die Verarbeitungseinheit (21) und der Speicher (22) bilden eine Steuer- und Recheneinheit, wie sie in Fig. 9 gezeigt ist.

Die Verarbeitungseinheit (21) (engl.: *processing unit)* kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (11, 32, 33) und/oder eine oder mehrere Benutzerschnittstellen (12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

Das Computersystem der vorliegenden Offenbarung kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

## Patentansprüche

1. Computer-implementiertes Verfahren umfassend:
- Empfangen mindestens eines Bildes (Iᵢ) eines Untersuchungsbereichs eines Untersuchungsobjekts,
- Erzeugen einer Vielzahl an Teilbildern (PI₂₁, PI₂₂, PI₂₃, PI₂₄, PI₂₅, PI₂₆) auf Basis des mindestens einen empfangenen Bildes (Iᵢ), wobei jedes Teilbild einen Teilbereich des Untersuchungsbereichs des Untersuchungsobjekts repräsentiert, wobei sich Teilbereiche, die von verschiedenen Teilbildern repräsentiert werden, teilweise aber nicht vollständig überlappen,
- für jedes erzeugte Teilbild (PI₂₁, PI₂₂, PI₂₃, PI₂₄, PI₂₅, PI₂₆): Erzeugen eines synthetischen Teilbildes (SI₂₁, SI₂₂, SI₂₃, SI₂₄, SI₂₅, SI₂₆) zumindest anteilig auf Basis des erzeugten Teilbildes (PI₂₁, PI₂₂, PI₂₃, PI₂₄, PI₂₅, PI₂₆),
- Ermitteln von Farbwerten korrespondierender Bildelemente synthetischer Teilbilder, wobei korrespondierende Bildelemente denselben Teilbereich des Untersuchungsbereichs repräsentieren,
- Ermitteln eines Streuungsmaßes der Farbwerte korrespondierender Bildelemente,
- Ermitteln eines Vertrauenswertes auf Basis des Streuungsmaßes,
- Ausgeben des Vertrauenswertes.

2. Verfahren gemäß Anspruch 1, wobei für jedes Tupel korrespondierender Bildelemente der synthetischen Teilbilder (SI₂₁, SI₂₂, SI₂₃, SI₂₄, SI₂₅, SI₂₆) ein Vertrauenswert ermittelt wird.

3. Verfahren gemäß einem der Ansprüche 1 oder 2, wobei das Streuungsmaß eine Streubreite, eine Standardabweichung, eine Varianz, eine Summe von Abweichungsquadraten, ein Variationskoeffizient, eine mittlere absolute Abweichung, ein Quantilsabstand, ein Interquantilsabstand, ein mittlerer absoluter Abstand von einem Median, ein Median absoluter Abweichungen und/oder eine geometrische Standardabweichung der Farbwerte korrespondierender Bildelemente ist oder davon abgeleitet ist.

4. Verfahren gemäß einem der Ansprüche 1 bis 3, wobei es für jedes Teilbild mindestens ein anderes Teilbild mit mindestens einem gemeinsamen Bildelement und mindestens einem unterschiedlichen Bildelement gibt.

5. Verfahren gemäß einem der Ansprüche 1 bis 4, wobei es zu jedem Bildelement des mindestens einen empfangenen Bildes (Iᵢ) eine Mehrzahl an Teilbildern gibt, die dieses Bildelement ebenfalls umfassen, wobei sich jedes Teilbild der Mehrzahl an Teilbildern von jedem anderen Teilbild der Mehrzahl an Teilbildern durch mindestens ein anderes Bildelement unterscheidet.

6. Verfahren gemäß einem der Ansprüche 1 bis 5, wobei das Erzeugen der Vielzahl an Teilbildern (PI₂₁, PI₂₂, PI₂₃, PI₂₄, PI₂₅, PI₂₆) umfasst:
- Erzeugen einer Mehrzahl von Kopien (I₁, I₂, I₃, I₄) des mindestens einen empfangenen Bildes (Iᵢ),
- Aufteilen jeder Kopie in Teilbilder, wobei sich alle Teilbilder aller Kopien voneinander unterscheiden.

7. Verfahren gemäß einem der Ansprüche 1 bis 6, wobei das Erzeugen der Vielzahl an Teilbildern (PI₂₁, PI₂₂, PI₂₃, PI₂₄, PI₂₅, PI₂₆) umfasst:
- Erzeugen einer Mehrzahl von Kopien (I₁, I₂, I₃, I₄) des mindestens einen empfangenen Bildes (Iᵢ),
- Aufteilen jeder Kopie in Teilbilder durch Schnitte, wobei die Schnitte bei jeder Kopie anders durch die Kopie verlaufen.

8. Verfahren gemäß einem der Ansprüche 1 bis 7, ferner umfassend:
- Erzeugen eines vereinten synthetischen Bildes (S) auf Basis der synthetischen Teilbilder (SI₂₁, SI₂₂, SI₂₃, SI₂₄, SI₂₅, SI₂₆), wobei das Erzeugen des vereinten synthetischen Bildes (S) das Kombinieren von Farbwerten korrespondierender Bildelemente der synthetischen Teilbilder (SI₂₁, SI₂₂, SI₂₃, SI₂₄, SI₂₅, SI₂₆) umfasst.

9. Verfahren gemäß Anspruch 8, wobei das Erzeugen des vereinten synthetischen Bildes (S) umfasst:
- für jedes Tupel korrespondierender Bildelemente der synthetischen Teilbilder (SI₂₁, SI₂₂, SI₂₃, SI₂₄, SI₂₅, SI₂₆): Ermitteln eines mittleren Farbwerts durch Mittelung der Farbwerte der korrespondierenden Bildelemente und Setzen des mittleren Farbwerts als Farbwert des korrespondierenden Bildelements des vereinten synthetischen Bildes (S).

10. Verfahren gemäß einem der Ansprüche 1 bis 9, ferner umfassend:
- Ausgeben des vereinten synthetischen Bildes (S) und/oder Übermitteln des vereinten synthetischen Bildes (S) an ein separates Computersystem.

11. Verfahren gemäß einem der Ansprüche 8 bis 9, ferner umfassend:
- Ermitteln eines Vertrauenswertes für einen oder mehrere Teilbereiche des vereinten synthetischen Bildes (S), und/oder für das gesamte vereinte synthetische Bild (S),
- Ausgeben des Vertrauenswertes.

12. Verfahren gemäß einem der Ansprüche 1 bis 11, wobei das Untersuchungsobjekt ein Mensch oder Tier, vorzugsweise ein Säugetier, ganz besonders bevorzugt ein Mensch ist.

13. Verfahren gemäß einem der Ansprüche 1 bis 12, wobei es sich bei dem mindestens einen empfangenen Bild (Iᵢ) um mindestens eine medizinische Aufnahme handelt und es sich bei jedem synthetischen Bild (SI₂₁, SI₂₂, SI₂₃, SI₂₄, SI₂₅, SI₂₆) und/oder bei dem vereinten synthetischen Bild (S) um eine synthetische medizinische Aufnahme handelt.

14. Verfahren gemäß einem der Ansprüche 1 bis 13,
wobei das mindestens eine empfangene Bild (Iᵢ) eine erste radiologische Aufnahme und eine zweite radiologische Aufnahme umfasst, wobei die erste radiologische Aufnahme den Untersuchungsbereich des Untersuchungsobjekts ohne Kontrastmittel oder nach Applikation einer ersten Menge eines Kontrastmittels repräsentiert und die zweite radiologische Aufnahme den Untersuchungsbereich des Untersuchungsobjekts nach Applikation einer zweiten Menge des Kontrastmittels repräsentiert,
wobei jedes synthetische Bild (SI₂₁, SI₂₂, SI₂₃, SI₂₄, SI₂₅, SI₂₆) und/oder das vereinte synthetische Bild (S) eine synthetische radiologische Aufnahme ist, wobei jedes synthetische Bild (SI₂₁, SI₂₂, SI₂₃, SI₂₄, SI₂₅, SI₂₆) und/oder das vereinte synthetische Bild (S) den Untersuchungsbereich des Untersuchungsobjekts nach Applikation einer dritten Menge des Kontrastmittels repräsentiert, wobei die zweite Menge verschieden von, vorzugweise größer ist als die erste Menge, und die dritte Menge verschieden von, vorzugsweise größer ist als die erste Menge und die zweite Menge.

15. Verfahren gemäß einem der Ansprüche 1 bis 13,
wobei das mindestens eine empfangene Bild (Iᵢ) eine erste radiologische Aufnahme und eine zweite radiologische Aufnahme umfasst, wobei die erste radiologische Aufnahme den Untersuchungsbereich des Untersuchungsobjekts in einer ersten Zeitspanne vor oder nach Applikation eines Kontrastmittels repräsentiert und die zweite radiologische Aufnahme den Untersuchungsbereich des Untersuchungsobjekts in einer zweiten Zeitspanne nach Applikation des Kontrastmittels repräsentiert,
wobei jedes synthetische Bild (SI₂₁, SI₂₂, SI₂₃, SI₂₄, SI₂₅, SI₂₆) und/oder das vereinte synthetische Bild (S) eine synthetische radiologische Aufnahme ist, wobei jedes synthetische Bild (SI₂₁, SI₂₂, SI₂₃, SI₂₄, SI₂₅, SI₂₆) und/oder das vereinte synthetische Bild (S) den Untersuchungsbereich des Untersuchungsobjekts in einer dritten Zeitspanne nach Applikation des Kontrastmittels repräsentiert, wobei die zweite Zeitspanne vorzugsweise zeitlich auf die erste Zeitspanne folgt und die dritte Zeitspanne vorzugsweise zeitlich auf die zweite Zeitspanne folgt.

16. Computersystem (10) umfassend
- eine Empfangseinheit (11),
- eine Steuer- und Recheneinheit (12) und
- eine Ausgabeeinheit (13),
wobei die Steuer- und Recheneinheit (12) konfiguriert ist,
- die Empfangseinheit (11) zu veranlassen, mindestens ein Bild eines Untersuchungsbereichs eines Untersuchungsobjekts zu empfangen,
- eine Vielzahl an Teilbildern auf Basis des mindestens einen empfangenen Bildes zu erzeugen, wobei jedes Teilbild einen Teilbereich des Untersuchungsbereichs des Untersuchungsobjekts repräsentiert, wobei sich Teilbereiche, die von verschiedenen Teilbildern repräsentiert werden, teilweise aber nicht vollständig überlappen.
- für jedes erzeugte Teilbild ein synthetisches Teilbild zumindest anteilig auf Basis des erzeugten Teilbildes zu erzeugen,
- Farbwerte korrespondierender Bildelemente synthetischer Teilbilder zu ermitteln, wobei korrespondierende Bildelemente denselben Teilbereich des Untersuchungsbereichs repräsentieren,
- ein Streuungsmaß der Farbwerte korrespondierender Bildelemente zu ermitteln,
- einen Vertrauenswertes auf Basis des Streuungsmaßes zu ermitteln,
- die Ausgabeeinheit (13) zu veranlassen, den Vertrauenswert auszugeben.

17. Computerlesbares Speichermedium umfassend ein Computerprogramm (40), das, wenn es in einen Arbeitsspeicher (22) eines Computersystems (10) geladen wird, das Computersystem (10) veranlasst, folgende Schritte auszuführen:
- Empfangen mindestens eines Bildes (Iᵢ) eines Untersuchungsbereichs eines Untersuchungsobjekts,
- Erzeugen einer Vielzahl an Teilbildern (PI₂₁, PI₂₂, PI₂₃, PI₂₄, PI₂₅, PI₂₆) auf Basis des mindestens einen empfangenen Bildes (Iᵢ), wobei jedes Teilbild einen Teilbereich des Untersuchungsbereichs des Untersuchungsobjekts repräsentiert, wobei sich Teilbereiche, die von verschiedenen Teilbildern repräsentiert werden, teilweise aber nicht vollständig überlappen,
- für jedes erzeugte Teilbild (PI₂₁, PI₂₂, PI₂₃, PI₂₄, PI₂₅, PI₂₆): Erzeugen eines synthetischen Teilbildes (SI₂₁, SI₂₂, SI₂₃, SI₂₄, SI₂₅, SI₂₆) zumindest anteilig auf Basis des erzeugten Teilbildes (PI₂₁, PI₂₂, PI₂₃, PI₂₄, PI₂₅, PI₂₆),
- Ermitteln von Farbwerten korrespondierender Bildelemente synthetischer Teilbilder, wobei korrespondierende Bildelemente denselben Teilbereich des Untersuchungsbereichs repräsentieren,
- Ermitteln eines Streuungsmaßes der Farbwerte korrespondierender Bildelemente,
- Ermitteln eines Vertrauenswertes auf Basis des Streuungsmaßes,
- Ausgeben des Vertrauenswertes.

## Claims

1. Computer-implemented method comprising:
- Receiving at least one image (Iᵢ) of an examination area of an examination object,
- Generating a plurality of sub-images (PI₂₁, PI₂₂, PI₂₃, PI₂₄, PI₂₃, PI₂₆) based on the at least one received image (Iᵢ), wherein each sub-image represents a sub-region of the examination area of the examination object, wherein sub-regions represented by different sub-images partially but not completely overlap,
- For each generated sub-image (PI₂₁, PI₂₂, PI₂₃, PI₂₃, PI₂₃, PI₂₆): generating a synthetic sub-image (SI₂₁, SI₂₂, SI₂₃, SI₂₄, SI₂₅, SI₂₆) at least proportionally on the basis of the generated sub-image (PI₂₁, PI₂₂, PI₂₃, PI₂₄, PI₂₅, PI₂₆),
- Determining color values of corresponding image elements of synthetic sub-images, wherein corresponding image elements represent the same sub-area of the examination area,
- Determining a scattering measure of the color values of corresponding image elements,
- Determining a confidence value on the basis of the scattering measure,
- Outputting the confidence value.

2. The method according to claim 1, wherein a confidence value is determined for each tuple of corresponding image elements of the synthetic sub-images (SI₂₁, SI₂₂, SI₂₃, SI₂₄, SI₂₃, SI₂₆).

3. The method according to any one of claims 1 or 2, wherein the scattering measure is a scatter width, a standard deviation, a variance, a sum of squares of deviation, a coefficient of variation, a mean absolute deviation, a quantile distance, an interquantile distance, a mean absolute distance from a median, a median of absolute deviations and/or a geometric standard deviation of the color values of corresponding image elements, or is derived therefrom.

4. The method according to any one of claims 1 to 3, wherein for each sub-image there is at least one other sub-image having at least one common image element and at least one different image element.

5. The method according to any one of claims 1 to 4, wherein for each image element of the at least one received image (Iᵢ) there is a plurality of sub-images which also comprise this image element, wherein each sub-image of the plurality of sub-images differs from each other sub-image of the plurality of sub-images by at least one other image element.

6. The method according to any one of claims 1 to 5, wherein generating the plurality of sub-images (PI₂₁, PI₂₂, PI₂₃, PI₂₄, PI₂₅, PI₂₆) comprises:
- Generating a plurality of copies (I₁, I₂, I₃, I₄) of the at least one received image (Iᵢ),
- Dividing each copy into sub-images, wherein all sub-images of all copies differ from each other.

7. The method according to any one of claims 1 to 6, wherein generating the plurality of sub-images (PI₂₁, PI₂₂, PI₂₃, PI₂₄, PI₂₅, PI₂₆) comprises:
- Generating a plurality of copies (I₁, I₂, I₃, I₄) of the at least one received image (Iᵢ),
- Dividing each copy into sub-images by cuts, wherein the cuts run differently through the copy for each copy.

8. The method according to any one of claims 1 to 7, further comprising:
- Generating a combined synthetic image (S) based on the synthetic sub-images (SI₂₁, SI₂₂, SI₂₃, SI₂₄, SI₂₅, SI₂₆), wherein generating the combined synthetic image (S) comprises combining color values of corresponding image elements of the synthetic sub-images (SI₂₁, SI₂₂, SI₂₃, SI₂₄, SI₂₅, SI₂₆).

9. The method according to claim 8, wherein generating the combined synthetic image (S) comprises:
- For each tuple of corresponding image elements of the synthetic sub-images (SI₂₁, SI₂₂, SI₂₃, SI₂₄, SI₂₅, SI₂₆): determining a mean color value by averaging the color values of the corresponding image elements and setting the mean color value as the color value of the corresponding image element of the combined synthetic image (S).

10. The method according to any one of claims 1 to 9, further comprising:
- Outputting the combined synthetic image (S) and/or transmitting the combined synthetic image (S) to a separate computer system.

11. The method according to any one of claims 8 to 9, further comprising:
- Determining a confidence value for one or more sub-regions of the combined synthetic image (S), and/or for the entire combined synthetic image (S),
- Outputting the confidence value.

12. The method according to any one of claims 1 to 11, wherein the examination object is a human or animal, preferably a mammal, most preferably a human.

13. The method according to any one of claims 1 to 12, wherein the at least one received image (Iᵢ) is at least one medical image and each synthetic image (SI₂₁, SI₂₂, SI₂₃, SI₂₄, SI₂₅, SI₂₆) and/or the combined synthetic image (S) is a synthetic medical image.

14. The method according to any one of claims 1 to 13, wherein the at least one received image (Iᵢ) comprises a first radiological image and a second radiological image, wherein the first radiological image represents the examination area of the examination object without contrast agent or after application of a first amount of a contrast agent and the second radiological image represents the examination area of the examination object after application of a second amount of the contrast agent, wherein each synthetic image (SI₂₁, SI₂₂, SI₂₃, SI₂₄, SI₂₅, SI₂₆) and/or the combined synthetic image (S) is a synthetic radiological image, wherein each synthetic image (SI₂₁, SI₂₂, SI₂₃, SI₂₄, SI₂₃, SI₂₆) and/or the combined synthetic image (S) represents the examination area of the examination object after application of a third amount of the contrast agent, wherein the second amount is different from, preferably greater than, the first amount, and the third amount is different from, preferably greater than, the first amount and the second amount.

15. The method according to any one of claims 1 to 13, wherein the at least one received image (Iᵢ) comprises a first radiological image and a second radiological image, wherein the first radiological image represents the examination area of the examination object in a first time period before or after application of a contrast agent and the second radiological image represents the examination area of the examination object in a second time period after application of the contrast agent, wherein each synthetic image (SI₂₁, SI₂₂, SI₂₃, SI₂₄, SI₂₅, SI₂₆) and/or the combined synthetic image (S) is a synthetic radiological image, wherein each synthetic image (SI₂₁, SI₂₂, SI₂₃, SI₂₄, SI₂₃, SI₂₆) and/or the combined synthetic image (S) represents the examination area of the examination object in a third time period after application of the contrast agent, wherein the second time period preferably follows the first time period in time and the third time period preferably follows the second time period in time.

16. Computer system (10) comprising
- a receiving unit (11),
- a control and computing unit (12) and
- an output unit (13),
wherein the control and computing unit (12) is configured
- to cause the receiving unit (11) to receive at least one image of an examination area of an examination object,
- generating a plurality of sub-images based on the at least one received image, wherein each sub-image represents a sub-area of the examination area of the examination object, sub-areas represented by different sub-images partially but not completely overlapping,
- generating a synthetic sub-image for each generated sub-image at least proportionally on the basis of the generated sub-image,
- determining color values of corresponding image elements of synthetic sub-images, wherein corresponding image elements represent the same sub-region of the examination area,
- determining a scattering measure of the color values of corresponding image elements,
- determining a confidence value on the basis of the scattering measure,
- to cause the output unit (13) to output the confidence value.

17. A computer readable storage medium comprising a computer program (40) which, when loaded into a working memory (22) of a computer system (10), causes the computer system (10) to perform the following steps:
- Receiving at least one image (Iᵢ) of an examination area of an examination object,
- Generating a plurality of sub-images (PI₂₁, PI₂₂, PI₂₃, PI₂₄, PI₂₃, PI₂₆) based on the at least one received image (Iᵢ), wherein each sub-image represents a sub-area of the examination area of the examination object, sub-areas represented by different sub-images partially but not completely overlapping each other,
- For each generated sub-image (PI₂₁, PI₂₂, PI₂₃, PI₂₃, PI₂₃, PI₂₆): generating a synthetic sub-image (SI₂₁, SI₂₂, SI₂₃, SI₂₄, SI₂₅, SI₂₆) at least proportionally on the basis of the generated sub-image (PI₂₁, PI₂₂, PI₂₃, PI₂₄, PI₂₅, PI₂₆),
- Determining color values of corresponding image elements of synthetic sub-images, wherein corresponding image elements represent the same sub-area of the examination area,
- Determining a scattering measure of the color values of corresponding image elements,
- Determining a confidence value on the basis of the scattering measure,
- Outputting the confidence value.

## Revendications

1. Procédé mis en œuvre par ordinateur comprenant :
- Réception d'au moins une image (Iᵢ) d'une zone d'examen d'un objet d'examen,
- générer une pluralité de sous-images (PI₂₁, PI₂₂, PI₂₃, PI₂₄, PI₂₅, PI₂₆) sur la base de ladite au moins une image reçue (Iᵢ), chaque sous-image représentant une sous-zone de la zone d'examen de l'objet d'examen, dans laquelle des sous-zones représentées par différentes sous-images se chevauchent partiellement mais pas complètement,
- pour chaque sous-image générée (PI₂₁, PI₂₂, PI₂₃, PI₂₄, PI₂₅, PI₂₆) : générer une sous-image synthétique (SI₂₁, SI₂₂, SI₂₃, SI₂₄, SI₂₅, SI₂₆) au moins en partie sur la base de la sous-image générée (PI₂₁, PI₂₂, PI₂₃, PI₂₄, PI₂₅, PI₂₆),
- déterminer des valeurs de couleur d'éléments d'image correspondants de sous-images synthétiques, dans laquelle les éléments d'image correspondants représentent la même sous-zone de la zone d'examen,
- déterminer une mesure de dispersion des valeurs de couleur des éléments d'image correspondants,
- déterminer une valeur de confiance basée sur la mesure de dispersion,
- sortir la valeur de confiance.

2. Procédé selon la revendication 1, dans lequel une valeur de confiance est déterminée pour chaque tuple d'éléments d'image correspondants des sous-images synthétiques (SI₂₁, SI₂₂, SI₂₃, SI₂₄, SI₂₅, SI₂₆).

3. Procédé selon l'une des revendications 1 ou 2, dans lequel la mesure de dispersion est ou est dérivée d'une largeur de dispersion, d'un écart type, d'une variance, d'une somme de carrés d'écart, d'un coefficient de variation, d'un écart absolu moyen, d'une distance de quantile, d'une distance d'interquantile, d'une distance absolue moyenne par rapport à une médiane, d'une médiane d'écarts absolus et/ou d'un écart type géométrique des valeurs de couleur des éléments d'image correspondants.

4. Procédé selon l'une quelconque des revendications 1 à 3, dans lequel, pour chaque sous-image, il existe au moins une autre sous-image ayant au moins un pixel commun et au moins un pixel différent.

5. Procédé selon l'une quelconque des revendications 1 à 4, dans lequel, pour chaque pixel de ladite au moins une image reçue (Iᵢ), il existe une pluralité de sous-images comprenant également ce pixel, chaque sous-image de ladite pluralité de sous-images se distinguant de chaque autre sous-image de ladite pluralité de sous-images par au moins un pixel différent.

6. Procédé selon l'une quelconque des revendications 1 à 5, dans lequel la génération de la pluralité de sous-images (PI₂₁, PI₂₂, PI₂₃, PI₂₄, PI₂₅, PI₂₆) comprend :
- générer une pluralité de copies (I₁, I₂, I₃, I₄) de ladite au moins une image reçue (Iᵢ),
- diviser chaque copie en sous-images dans lesquelles toutes les sous-images de toutes les copies sont différentes les unes des autres.

7. Procédé selon l'une quelconque des revendications 1 à 6, dans lequel la génération de la pluralité de sous-images (PI₂₁, PI₂₂, PI₂₃, PI₂₄, PI₂₅, PI₂₆) comprend :
- générer une pluralité de copies (I₁, I₂, I₃, I₄) de ladite au moins une image reçue (Iᵢ),
- diviser chaque copie en sous-images par des coupes, dans lesquelles les coupes passent différemment à travers la copie pour chaque copie.

8. Procédé selon l'une quelconque des revendications 1 à 7, comprenant en outre :
- la génération d'une image synthétique unifiée (S) sur la base des sous-images synthétiques (SI₂₁, SI₂₂, SI₂₃, SI₂₄, SI₂₅, SI₂₆), dans lequel la génération de l'image synthétique unifiée (S) comprend la combinaison des valeurs de couleur des éléments d'image correspondants des sous-images synthétiques (SI₂₁, SI₂₂, SI₂₃, SI₂₄, SI₂₅, SI₂₆).

9. Procédé selon la revendication 8, dans lequel la génération de l'image synthétique unifiée (S) comprend :
- pour chaque tuple d'éléments d'image correspondants des sous-images synthétiques (SI₂₁, SI₂₂, SI₂₃, SI₂₄, SI₂₅, SI₂₆) : déterminer une valeur de couleur moyenne en faisant la moyenne des valeurs de couleur des éléments d'image correspondants et définir la valeur de couleur moyenne comme valeur de couleur de l'élément d'image correspondant de l'image synthétique fusionnée (S).

10. Procédé selon l'une quelconque des revendications 1 à 9, comprenant en outre :
- sortir l'image synthétique fusionnée (S) et/ou transmettre l'image synthétique fusionnée (S) à un système informatique séparé.

11. Procédé selon l'une des revendications 8 à 9, comprenant en outre :
- déterminer une valeur de confiance pour une ou plusieurs parties de l'image synthétique unifiée (S), et/ou pour l'ensemble de l'image synthétique unifiée (S),
- sortir la valeur de confiance.

12. Procédé selon l'une quelconque des revendications 1 à 11, dans lequel l'objet d'examen est un être humain ou un animal, de préférence un mammifère, de manière tout à fait préférée un être humain.

13. Procédé selon l'une quelconque des revendications 1 à 12, dans lequel ladite au moins une image reçue (Iᵢ) est au moins une image médicale et chaque image synthétique (SI₂₁, SI₂₂, SI₂₃, SI₂₄, SI₂₅, SI₂₆) et/ou l'image synthétique combinée (S) est une image médicale synthétique.

14. Procédé selon l'une quelconque des revendications 1 à 13, dans lequel ladite au moins une image reçue (Iᵢ) comprend une première image radiologique et une seconde image radiologique, ladite première image radiologique représentant la zone d'examen de l'objet examiné sans agent de contraste ou après application d'une première quantité d'un agent de contraste et ladite seconde image radiologique représentant la zone d'examen de l'objet examiné après application d'une seconde quantité de l'agent de contraste, dans lequel chaque image synthétique (SI₂₁, SI₂₂, SI₂₃, SI₂₄, SI₂₅, SI₂₆) et/ou l'image synthétique combinée (S) est une image radiologique synthétique, dans laquelle chaque image synthétique (SI₂₁, SI₂₂, SI₂₃, SI₂₄, SI₂₅, SI₂₆) et/ou l'image synthétique combinée (S) représente la zone d'examen de l'objet examiné après application d'une troisième quantité de l'agent de contraste, la deuxième quantité étant différente de, de préférence supérieure à la première quantité, et la troisième quantité étant différente de, de préférence supérieure à la première quantité et à la deuxième quantité.

15. Procédé selon l'une quelconque des revendications 1 à 13, dans lequel ladite au moins une image reçue (Iᵢ) comprend une première image radiologique et une seconde image radiologique, ladite première image radiologique représentant la zone d'examen du sujet dans une première période de temps avant ou après l'application d'un agent de contraste et ladite seconde image radiologique représentant la zone d'examen du sujet dans une seconde période de temps après l'application de l'agent de contraste, dans lequel chaque image synthétique (SI₂₁, SI₂₂, SI₂₃, SI₂₄, SI₂₅, SI₂₆) et/ou l'image synthétique unifiée (S) est une image radiologique synthétique, dans laquelle chaque image synthétique (SI₂₁, SI₂₂, SI₂₃, SI₂₄, SI₂₅, SI₂₆) et/ou l'image synthétique unifiée (S) représente la zone d'examen de l'objet d'étude dans une troisième période de temps après l'application du produit de contraste, la deuxième période de temps suivant de préférence dans le temps la première période de temps et la troisième période de temps suivant de préférence dans le temps la deuxième période de temps.

16. Système informatique (10) comprenant
- une unité de réception (11),
- une unité de commande et de calcul (12) et
- une unité de sortie (13),
dans lequel l'unité de commande et de calcul (12) est configurée,
- d'amener l'unité de réception (11) à recevoir au moins une image d'une zone d'examen d'un objet d'examen,
- générer une pluralité de sous-images sur la base de ladite au moins une image reçue, chaque sous-image représentant une sous-zone de la zone d'examen de l'objet d'examen, dans laquelle les sous-zones représentées par différentes sous-images se chevauchent partiellement mais pas complètement,
- pour chaque sous-image générée, générer une sous-image synthétique au moins en partie sur la base de la sous-image générée,
- déterminer les valeurs de couleur des éléments d'image correspondants des sous-images synthétiques, dans lesquelles les éléments d'image correspondants représentent la même sous-zone de la zone d'examen,
- déterminer une mesure de dispersion des valeurs de couleur des éléments d'image correspondants,
- déterminer une valeur de confiance sur la base de la mesure de dispersion,
- faire en sorte que l'unité de sortie (13) émette la valeur de confiance.

17. Support de stockage lisible par ordinateur comprenant un programme d'ordinateur (40) qui, lorsqu'il est chargé dans une mémoire de travail (22) d'un système informatique (10), amène le système informatique (10) à exécuter les étapes suivantes :
- Réception d'au moins une image (Iᵢ) d'une zone d'examen d'un objet d'examen,
- générer une pluralité de sous-images (PI₂₁, PI₂₂, PI₂₃, PI₂₄, PI₂₃, PI₂₆) sur la base de ladite au moins une image reçue (Iᵢ), chaque sous-image représentant une sous-zone de la zone d'examen de l'objet d'examen, dans laquelle des sous-zones représentées par différentes sous-images se chevauchent partiellement mais pas complètement,
- pour chaque sous-image générée (PI₂₁, PI₂₂, PI₂₃, PI₂₄, PI₂₅, PI₂₆) : générer une sous-image synthétique (SI₂₁, SI₂₂, SI₂₃, SI₂₄, SI₂₅, SI₂₆) au moins en partie sur la base de la sous-image générée (PI₂₁, PI₂₂, PI₂₃, PI₂₄, PI₂₅, PI₂₆),
- déterminer des valeurs de couleur d'éléments d'image correspondants de sous-images synthétiques, dans laquelle les éléments d'image correspondants représentent la même sous-zone de la zone d'examen,
- déterminer une mesure de dispersion des valeurs de couleur des éléments d'image correspondants,
- déterminer une valeur de confiance basée sur la mesure de dispersion,
- sortir la valeur de confiance.
